# EUROPEAN PATENT APPLICATION

(11) **EP 2 719 397 A1**
(43) Date of publication of application: **16.04.2014**
(21) Application number: 13168453.2
(22) Date of filing: 27.02.2007
(51) Int. Cl.: A61K 39/40, A61P 31/04

(54) **Use of alpha-toxin for treating and preventing staphylococcus infections**

(30) Priority: 12.06.2006 US 812598 P; 18.12.2006 US 875363 P
(62) Divisional of application: 07751817.3
(71) Applicant: GlaxoSmithKline Biologicals SA, 1330 Rixensart (BE)
(72) Inventor: Taylor, Kimberly L, Bethesda, MD Maryland 20814 (US); Fattom, Ali I, Rockville, MD Maryland 20852 (US)
(74) Representative: Johnston, Caroline Louise

(57) **Abstract**

Vaccines comprising an *S. aureus* alpha-toxin antigen and a pharmaceutically acceptable carrier are provided, and are useful for treating and preventing infections. The *S*. *aureus* alpha-toxin antigen may contain at least two alterations that reduce its toxicity and/or may be conjugated to or co-administered with another bacterial antigen. The vaccines may comprise one or more other bacterial antigens.

## Description

### RELATED APPLICATIONS

This application claims the benefits of priority to U.S. provisional application 60/875,363, filed December 18, 2006, and U.S. provisional application 60/812,598, filed June 12, 2006, the entire contents of which are incorporated herein by reference.

### BACKGROUND OF THE INVENTION

This invention relates to the treatment and prevention of bacterial infections. In particular, the invention provides compositions and methods for treating and preventing *Staphylococcus aureus (S. aureus)* and other bacterial infections, including infections associated with methicillin resistant *S*. *aureus* strains such as those that produce alpha-toxin.

*Staphylococcus aureus* bacteria, often referred to as "staph," *"Staph. aureus,"* or *"S. aureus,"* commonly colonize the nose and skin of healthy humans. Approximately 20-30% of the population is colonized with *S*. *aureus* at any given time. These bacteria often cause minor infections, such as pimples and boils, in healthy individuals but also cause systemic infections. They are considered to be opportunistic pathogens. Normally, mucosal and epidermal barriers (skin) protect against *S*. *aureus* infections. Interruption of these natural barriers as a result of injuries - such as bums, trauma or surgical procedures - dramatically increases the risk of infection. Diseases that compromise the immune system (e.g., diabetes, end-stage renal disease, cancer) also increase the risk of infection. Opportunistic *S*. *aureus* infections can become quite serious, causing endocarditis, bacteremia and osteomyelitis, which often result in severe morbidity or mortality.

*S. aureus* expresses a number of virulence factors including capsular polysaccharides and protein toxins. One important virulence factor is alpha-toxin (alpha-hemolysin), a pore-forming and hemolytic exoprotein produced by most pathogenic strains of *S*. *aureus.* Studies have shown that human white blood cells, erythrocytes, platelets and endothelial cells are particularly susceptible to the hemolytic effects of alpha-toxin. Such studies establish the relevance of alpha-toxin to human pathophysiology.

Anti-alpha-toxin immunity has been shown to protect against the toxin's detrimental effects, but designing vaccines against alpha-toxin remains a significant challenge. This is so because the need to induce a protective immune response must be balanced against the need to avoid causing illness related to the toxin's biological activity. While chemical and molecular modifications of alpha-toxin reportedly can reduce its toxicity, no single reported modification entirely eliminates the toxicity of alpha-toxin. Additionally, there exists a real risk that modified alpha-toxins might revert to their earlier more toxic state. This makes any singly modified alpha-toxin unsuitable for use in a human vaccine.

Accordingly, there remains a need in the art for compositions and methods that can safely confer immunity to alpha-toxin and *S*. *aureus* bacteria. The present invention meets this and other needs.

### SUMMARY OF THE INVENTION

The present invention provides vaccines for treating *S*. *aureus* infections, methods of treating and preventing *S*. *aureus* infections, antibody compositions (including) intravenous immunoglobulin (IVIG) compositions, and methods of making antibody compositions.

In one embodiment, there is provided a pentavalent *Staphylococcal* antigen composition comprising (i) an *S. aureus* Type 5 antigen, (ii) an *S*. *aureus* Type 8 antigen, (iii) an *S*. *aureus* 336 antigen, (iv) an *S. aureus* alpha-toxin antigen and (v) a *Staphylococcal* leukocidin antigen. In one embodiment, at least one of the *Staphylococcal* antigens is a protective antigen. In one embodiment, the *S*. *aureus* alpha-toxin antigen is conjugated to at least one of the Type 5 antigen, Type 8 antigen, 336 antigen, or leukocidin antigen.

In one embodiment, the alpha-toxin antigen contains at least two alterations, relative to wild-type *S*. *aureus* alpha-toxin, that reduce its toxicity. In one embodiment, the *Staphylococcal* leukocidin antigen is selected from the group consisting of Panton-Valentine Leukocidin (PVL) antigen subunits and gamma-hemolysin subunit antigens. In one embodiment, the *Staphylococcal* leukocidin antigen is selected from the group consisting of (i) a LukF-PV subunit of *S*. *aureus* PVL, (ii) a LukS-PV subunit of *S*. *aureus* PVL, (iii) a HlgA *S*. *aureus* gamma-hemolysin subunit, (iv) a HlgB *S*. *aureus* gamma-hemolysin subunit; (v) a HlgC *S*. *aureus* gamma-hemolysin subunit, (vi) LukD from *S*. *aureus,* (vii) LukE from *S*. *aureus,* (viii) LukM from *S*. *aureus,* (ix) a LukF'-PV subunit of *S. aureus* PVL, (x) a LukF-I subunit from *S*. *intermedius;* and (xi) a LukS-I subunit from *S. intermedius.* In one embodiment, the composition further comprises one or more additional bacterial antigens, such as a *Staphylococcal* antigen selected from the group consisting of *S*. *epidermidis* PS1, *S*. *epidermidis* GP1, lipoteichoic acid (LTA) and microbial surface components recognizing adhesive matrix molecule (MSCRAMM) proteins, and combinations thereof.

In another embodiment, there is provided a composition comprising an *S*. *aureus* alpha-toxin antigen and a pharmaceutically acceptable carrier, wherein the alpha-toxin antigen contains at least two alterations, relative to wild-type *S*. *aureus* alpha-toxin, that reduce its toxicity. In one embodiment, at least one of the alterations is a chemical alteration. In another embodiment, at least one of the alterations is molecular alteration. In yet another embodiment, at least one of the alterations is a chemical alteration and at least one is a molecular alteration.

In one embodiment, a molecular alteration is a substitution, insertion or deletion in the amino acid sequence of wild-type *S*. *aureus* alpha-toxin. In one embodiment, the molecular alteration is a substitution in the amino acid sequence of wild-type *S*. *aureus* alpha-toxin. In one embodiment, the substitution occurs at a location corresponding to His-35 of wild-type *S*. *aureus* alpha-toxin. In one embodiment, the substitution is a substitution of Arg, Lys, Ala, Leu, or Glu for His. In one embodiment, a molecular alteration is a substitution, insertion or deletion in the amino latch domain of wild-type *S*. *aureus* alpha-toxin. In one embodiment, the molecular alteration is a deletion in the amino latch domain of wild-type *S*. *aureus* alpha-toxin. In one embodiment, the molecular alteration is a deletion in the stem domain of wild-type *S*. *aureus* alpha-toxin.

In another embodiment, there is provided a composition comprising (i) an *S. aureus* alpha-toxin antigen and (ii) one or more additional bacterial antigens other than the *S*. *aureus* alpha-toxin antigen. In one embodiment, at least one of the one or more additional bacterial antigens is an additional *Staphylococcal* antigen selected from the group consisting of *S*. *aureus* Type 5, *S*. *aureus* Type 8, *S*. *aureus* 336, *Staphylococcal* leukocidin antigens, *S*. *epidermidis* PS1, *S*. *epidermidis* GP1, lipoteichoic acid (LTA) and microbial surface components recognizing adhesive matrix molecule (MSCRAMM) proteins, and combinations thereof. In one embodiment, the additional *Staphylococcal* antigen is a protective antigen. In one embodiment, the *S*. *aureus* alpha-toxin antigen is conjugated to at least one of the one or more additional bacterial antigens. In one embodiment, the alpha-toxin antigen contains at least two alterations, relative to wild-type *S. aureus* alpha-toxin, that reduce its toxicity.

In another embodiment, there is provided a method for treating or preventing *S*. *aureus* infection comprising administering to a subject in need thereof any of the aforementioned antigen compositions. In one embodiment, the method further comprises administering an agent selected from the group consisting of an antiinfective agent, an antibiotic agent, and an antimicrobial agent, such as vancomycin, lysostaphin or clindamycin. In one embodiment, the *S*. *aureus* infection is associated with a methicillin resistant *S*. *aureus.* In one embodiment, the methicillin resistant *S*. *aureus* produces alpha-toxin.

In another embodiment, there is provided a method of making a hyperimmune specific intravenous immunoglobulin (IVIG) preparation, comprising (i) administering to a subject any of the above-described compositions, (ii) harvesting plasma from the subject, and (iii) purifying an immunoglobulin from the subject.

In another embodiment, there is provided a pentavalent *Staphylococcal* antibody composition comprising (i) a first antibody that specifically binds to an *S*. *aureus* Type 5 antigen, (ii) a second antibody that specifically binds to an *S*. *aureus* Type 8 antigen, (iii) a third antibody that specifically binds to an *S*. *aureus* 336 antigen, (iv) a fourth antibody that specifically binds to an *S*. *aureus* alpha-toxin antigen and (v) a fifth antibody that specifically binds to an *Staphylococcal* leukocidin antigen. In one embodiment, at least one of the first through fifth antibodies is a monoclonal antibody. In one embodiment, at least one of the first through fifth antibodies is a neutralizing antibody. In one embodiment, the fifth antibody specifically binds to a *Staphylococcal* leukocidin antigen selected from the group consisting of Panton-Valentine Leukocidin (PVL) antigen subunits and gamma-hemolysin subunit antigens. In one embodiment, the fifth antibody specifically binds to a *Staphylococcal* leukocidin antigen selected from the group consisting of (i) a LukF-PV subunit of *S. aureus* PVL, (ii) a LukS-PV subunit of *S*. *aureus* PVL, (iii) a HlgA *S*. *aureus* gamma-hemolysin subunit, (iv) a HlgB *S*. *aureus* gamma-hemolysin subunit; (v) a HlgC *S*. *aureus* gamma-hemolysin subunit, (vi) LukD from *S*. *aureus,* (vii) LukE from *S*. *aureus,* (viii) LukM from *S*. *aureus,* (ix) a LukF'-PV subunit of *S. aureus* PVL, (x) a LukF-I subunit from *S*. *intermedius;* and (xi) a LukS-I subunit from *S. intermedius.*

In another embodiment, there is provided a protective antibody composition, comprising (i) a first antibody that specifically binds to an *S*. *aureus* alpha-toxin antigen and (ii) at least one second antibody that specifically binds to a bacterial antigen other than said *S*. *aureus* alpha-toxin antigen. In one embodiment, at least one of the first and second antibodies is a monoclonal antibody. In one embodiment, at least one of the first and second antibodies is a neutralizing antibody. In one embodiment, at least one of the at least one second antibody specifically binds to an additional *Staphylococcal* antigen selected from the group consisting of *S*. *aureus* Type 5, *S*. *aureus* Type 8, *S*. *aureus* 336*, Staphylococcal* leukocidin antigens, *S. epidermidis* PS1, *S*. *epidermidis* GP1, lipoteichoic acid (LTA) and microbial surface components recognizing adhesive matrix molecule (MSCRAMM) proteins. In one embodiment, at least one of the at least one second antibody specifically binds to a *Staphylococcal* leukocidin antigen selected from the group consisting of Panton-Valentine Leukocidin (PVL) antigen subunits and gamma-hemolysin subunit antigens. In one embodiment, at least one of the at least one second antibody specifically binds to a *Staphylococcal* leukocidin antigen selected from the group consisting of (i) a LukF-PV subunit of *S. aureus* PVL, (ii) a LukS-PV subunit of *S*. *aureus* PVL, (iii) a HlgA *S*. *aureus* gamma-hemolysin subunit, (iv) a HlgB *S*. *aureus* gamma-hemolysin subunit; (v) a HlgC *S*. *aureus* gamma-hemolysin subunit, (vi) LukD from *S*. *aureus,* (vii) LukE from *S*. *aureus,* (viii) LukM from *S*. *aureus,* (ix) a LukF'-PV subunit of *S. aureus* PVL, (x) a LukF-I subunit from *S*. *intermedius;* and (xi) a LukS-I subunit from *S. intermedius.* In one embodiment, the composition comprises a sub-optimal amount of said first antibody and a sub-optimal amount of said second antibody.

In one embodiment, the composition is prepared by a method comprising (a) administering (i) an *S. aureus* alpha-toxin antigen and (ii) one or more additional bacterial antigens other than said *S*. *aureus* alpha-toxin antigen to a human subject, (b) harvesting plasma from said subject, and (c) purifying immunoglobulin from said subject. In one embodiment, the method uses *S*. *aureus* alpha-toxin antigen conjugated to at least one of said one or more additional bacterial antigens. In one embodiment, the method uses *S*. *aureus* alpha-toxin antigen containing at least two alterations, relative to wild-type *S*. *aureus* alpha-toxin, that reduce its toxicity.

In one embodiment, the composition is prepared by a method comprising (a) screening a human subject that has not been administered an *S*. *aureus* alpha-toxin antigen and an additional bacterial antigen other than said *S*. *aureus* alpha-toxin antigen, (b) harvesting plasma from said subject, and (c) purifying immunoglobulin from said subject.

In another embodiment, there is provided a method for treating or preventing *S*. *aureus* infection, comprising administering to a subject in need thereof any of the aforementioned antibody compositions. In one embodiment, the method further comprises administering an agent selected from the group consisting of an antiinfective agent, an antibiotic agent, and an antimicrobial agent, such as vancomycin, lysostaphin or clindamycin. In one embodiment, the *S*. *aureus* infection is associated with methicillin resistant *S*. *aureus.* In one embodiment, the *S*. *aureus infection* produces alpha-toxin.

In another embodiment, there is provided a method of neutralizing *S*. *aureus* PVL infection comprising administering to a patient in need thereof a composition comprising (i) a *Staphylococcal* leukocidin antigen or (ii) an antibody that specifically binds to a *Staphylococcal* leukocidin antigen.

In another embodiment, there is provided a method of neutralizing *Staphylococcal* leukocidin infection comprising administering to a patient in need thereof a composition comprising (i) an *S. aureus* PVL antigen subunit or (ii) an antibody that specifically binds to an *S*. *aureus* PVL antigen subunit.

### BRIEF DESCRIPTION OF THE DRAWINGS

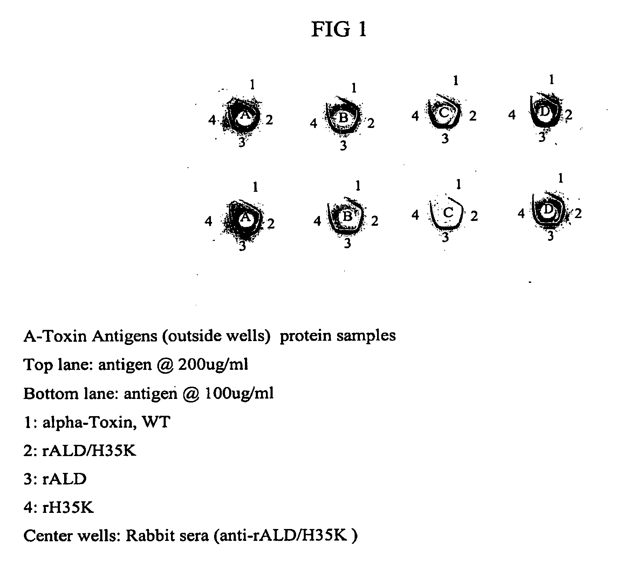

Figure 1: Immunodiffusion of alpha-toxin proteins with rabbit polyclonal anti-ALD/H35K

### DETAILED DESCRIPTION

The present invention provides vaccines for treating *S*. *aureus* infections, methods of treating and preventing *S*. *aureus* infections, antibody compositions (including IVIG compositions), and methods of making antibody compositions. In discussing these aspects of the invention, the use of "a," "an," and "the" means "one or more," unless otherwise specified.

It is commonly appreciated that bacterial polysaccharides (PS) are T-cell independent antigens and, as such, when administered alone do not elicit significant levels of antibodies in naïve populations and small children, *i*.*e*., do not trigger an anamnestic immune response. Similar to the vast majority of bacterial polysaccharides, *S*. *epidermidis* PS1 alone (unconjugated to protein) does not elicit a specific antibody immune response. However, by chemically conjugating polysaccharides to proteins (PR), the polysaccharides acquire properties of T-cell dependent antigens, such as immunological memory and long lasting IgG response. Suitability of proteins to function as protein carriers in the PS-PR conjugate vaccines is usually evaluated by measuring antibody responses specific to PS.

In the case of the PS1-rALD/H35K conjugate described herein, the protein carrier, rALD/H35K, is a clinically important antigen and antibodies specific to rALD/H35K can neutralize native alpha-toxin. Therefore, the magnitude of anti-alpha-toxin antibody response induced by PS1-rALD/H35K is of clinical importance.

### Vaccine Compositions

The invention provides vaccines that comprise an *S*. *aureus* alpha-toxin antigen. As used herein, *"S. aureus* alpha-toxin antigen" or "alpha-toxin antigen" refers to any molecule comprising an antigenic portion of *S*. *aureus* alpha-toxin, including full length *S*. *aureus* alpha-toxin and fragments thereof. Fragments of *S*. *aureus* alpha-toxin suitable for use in the present invention possess antigenic properties similar to wild-type *S. aureus* alpha-toxin. For example, such antigens induce antibodies that specifically bind to wild-type *S*. *aureus* alpha-toxin. The *S*. *aureus* alpha-toxin antigen may be about 5, 10, 15, 20, 25, 30, 35, 40, 45, 50, 60, 70, 80, 90, 100, 110, 120, 130, 140, 150, 160, 170, 180, 190, 200, 210, 220, 230, 240, 250, 260, 270, 280, 290 or 300 amino acids in length. The *S*. *aureus* alpha-toxin antigen may comprise about 5, 10, 15, 20, 25, 30, 35, 40, 45, 50, 60, 70, 80, 90, 100, 110, 120, 130, 140, 150, 160, 170, 180, 190, 200, 210, 220, 230, 240, 250, 260, 270, 280, or 290 consecutive amino acids of wild-type *S*. *aureus* alpha-toxin. Across its length, the *S. aureus* alpha-toxin antigen's amino acid sequence may be about 10, 20, 30, 40, 50, 60, 70,75, 80, 85,90, 95 or 100 percent identical to the amino acid sequence of wild-type S. *aureus* alpha-toxin (SEQ ID NO: 1) or a corresponding portion of *S*. *aureus* alpha-toxin.

The *S. aureus* alpha-toxin antigen may be a recombinant antigen, meaning that the antigen was made by recombinant DNA methodologies. Such recombinant DNA methodologies are well known in the art. Recombinant *S*. *aureus* alpha-toxin antigens are generally free from other proteins and cell components with which wild-type *S*. *aureus* alpha-toxin is associated in its native state (*i*.*e*., proteins and cell components present in *Staph.* cells). An exemplary recombinant host for making *S*. *aureus* alpha-toxin antigens is *E*. *coli.* The antigens can first be expressed in *E*. *coli* cells and then purified from *E*. *coli* using, for example, affinity column chromatography.

*S. aureus* alpha-toxin antigens useful in the present invention may comprise one or more amino acid insertions, substitutions or deletions relative to wild-type *S*. *aureus* alpha-toxin. For example, one or more amino acid residues within the *S*. *aureus* alpha-toxin sequence may be substituted by another amino acid of a similar polarity, which acts as a functional equivalent, resulting in a silent alteration. Substitutions within the antigen may be selected from other members of the class to which the amino acid belongs. For example, nonpolar (hydrophobic) amino acids include alanine, leucine, isoleucine, valine, proline, phenylalanine, tryptophan and methionine. Polar neutral amino acids include glycine, serine, threonine, cysteine, tyrosine, asparagine, and glutamine. Positively charged (basic) amino acids include arginine, lysine and histidine. Negatively charged (acidic) amino acids include aspartic acid and glutamic acid. Alternatively, non-conservative amino acid alterations may be made, including the alterations discussed in more detail below in the context of detoxifying *S*. *aureus* alpha-toxin antigens. Thus, in some embodiments, a non-conservative amino acid change is made to the *S*. *aureus* alpha-toxin antigen to detoxify it.

In accordance with the present invention, *S*. *aureus* alpha-toxin antigens are altered, relative to wild-type *S*. *aureus* alpha-toxin antigen, to reduce their toxicity. In one embodiment, the antigens contain at least two alterations, relative to the wild-type antigen. This embodiment minimizes toxicity and also reduces the risk that the antigen will revert to a more toxic state. For example, the antigens may contain 2, 3, 4, 5-10, 10-15, 15-20 or more alterations. The alterations may be "chemical alterations," may be "molecular alterations," or may be a combination of chemical and molecular alterations. For purposes of counting the number of alterations, the chemical or molecular modification of a single amino acid or a contiguous sequence of amino acids is considered a single alteration. Thus, the deletion, substitution or insertion of two or more contiguous amino acids is "a single alteration," as used herein.

"Chemical alteration" refers to a modification effected by chemical treatment of the *S. aureus* alpha-toxin antigen or conjugation of the *S*. *aureus* alpha-toxin antigen to another moiety. For example, chemical modification of histidines in *S*. *aureus* alpha-toxin with diethylpyrocarbonate is known to reduce alpha-toxin's hemolytic activity. Conjugation of *S. aureus* alpha-toxin to other molecules also reduces the alpha-toxin's hemolytic activity. In one embodiment, the other molecule is another bacterial antigen, such as a bacterial polysaccharide or a bacterial glycoprotein. The bacterial antigens may be *S*. *aureus* antigens or may be derived from other bacterial species. Exemplary bacterial antigens include *S*. *aureus* Type 5, *S*. *aureus* Type 8, *S*. *aureus 336, S. epidermidis* PS1, *S*. *epidermidis* GP1, leukocidins such as PVL (including the individual PVL subunits, LukS-PV and LukF-PV) and gamma-hemolysin subunits (HlgA, HlgB, and HlgC), LukD from *S. aureus,* LukE from *S*. *aureus,* LukM from *S*. *aureus,* LukF'-PV from *S*. *aureus,* a LukF-I subunit from *S*. *intermedius,* or a LukS-I subunit from *S*. *intermedius,* lipoteichoic acid (LTA) and microbial surface components recognizing adhesive matrix molecule (MSCRAMM) proteins. Thus, vaccines of the invention may comprise an alpha-toxin antigen-Type 5 conjugate, an alpha-toxin antigen-Type 8 conjugate, an alpha-toxin antigen-Type 336 conjugate, an alpha-toxin-PVL conjugate, an alpha-toxin antigen-PS 1 conjugate, an alpha-toxin antigen-GP1 conjugate, an alpha-Toxin LTA conjugate, or an alpha-toxin-MSCRAMM conjugate. Similarly, vaccines of the invention may comprise an alpha-toxin antigen that is altered and detoxified by conjugation to another molecule, such as another bacterial polysaccharide, another Gram-positive bacterial antigen or a Gram-negative bacterial antigen.

"Molecular alteration" refers to a modification in the amino acid sequence of *S*. *aureus* alpha-toxin. The modification may be an insertion, a deletion or a substitution of one or more amino acids. Molecular alterations may occur in any part of the *S*. *aureus* alpha-toxin. In one embodiment, the amino latch domain is molecularly modified. For example, a portion of the amino latch domain or the entire amino latch domain (Ala¹ - Val²⁰) may be deleted, thereby detoxifying the alpha-toxin antigen. In another embodiment, the stem domain (Lys¹¹⁰ - Tyr¹⁴⁸) is molecularly modified. For example, a portion of the stem domain or the entire stem domain may be deleted. In another embodiment, amino acid residues forming the triangle region (Pro¹⁰³ - Thr¹⁰⁹ and Val¹⁴⁹ - Asp¹⁵²) are molecularly modified. In another embodiment, the cap domain is molecularly modified. In another embodiment, the rim domain is molecularly modified. In another embodiment, one or more histidine residues are modified, such as His³⁵, His⁴⁸, His¹⁴⁴ and His²⁵⁹. Modification of His³⁵ is exemplary. For example, the modification may be a His³⁵Lys, His³⁵Arg, His³⁵Ala, His³⁵Leu or His³⁵Glu substitution. His³⁵Lys substitution is one particular embodiment. Other exemplary residues that may be modified include Asp²⁴, Lys³⁷, Lys⁵⁸, Asp¹⁰⁰,Ile¹⁰⁷, Glu¹¹¹, Met¹¹³, Asp¹²⁷ , Asp¹²⁸ , Gly¹³⁰, Gly¹³⁴, Lys¹⁴⁷, Gln¹⁵⁰, ASp¹⁵², Phe¹⁵³, Lys¹⁵⁴, Val¹⁶⁹, Asn¹⁷³, Arg²⁰⁰, Asn²¹⁴ and Leu²¹⁹.

Molecular alterations can be accomplished by methods well known in the art, including primer extension on a plasmid template using single stranded templates by the original Kunkel method (Kunkel, TA, Proc. Acad. Sci., USA, 82:488-492 (1985)) or double stranded DNA templates (Papworth et al., Strategies, 9(3):3-4 (1996)), and by PCR cloning (Braman,J. (ed.), IN VITRO MUTAGENESIS PROTOCOLS, 2nd ed. Humana Press, Totowa, NJ (2002), Ishii et al., Meth. Enzymol., 293, , 53-71 (1998), Kammann et al., Nucleic Acids Res.,17:5404 (1989), Hemsley et al, Nucleic Acids Res., 17:6545-6551 (1989), Giebel et al., Nucleic Acids Res., 18:4947 (1990), Landt et al., Gene, 96:125-128 (1990), Stemmer et al., BioTechniques, 13:214-220 (1992), Marini et al., Nucleic Acids Res., 21:2277-2278 (1993), and Weiner et al., Gene, 151:119-123 (1994)).

Methods of determining whether an alteration reduces the toxicity of an *S. aureus* alpha-toxin antigen are known in the art. Alpha-toxin permeabilizes membranes, causing rapid egress of cellular components. Accordingly, pore formation and death of nucleated cells can conveniently be registered by conventional dye exclusion tests, by measuring the uptake of a fluorescent dye such as propidium iodide or ethidium bromide, or by measuring ATP leakage. Techniques useful for measuring alpha-toxin toxicity include light or fluorescent microscopy, flow cytometry, and flourimetry.

Bernheimer described a hemolytic assay using erythrocytes to measure toxicity. (Bernheimer, A.W., Methods Enzymol., 165: 213-217 (1988)). The standard procedure for determining hemolytic titer is to add a suspension of erythrocytes to serially diluted toxin. The reciprocal of the dilution eliciting 50% lysis within 1 hour at room temperature gives the number of hemolytic units (HU), which can be expressed per milligram of protein. The specific activity of purified alpha-toxin is in the range of 40,000 HU/mg of protein, when assessed by addition of 1 volume of 2.5% erythrocyte suspension (2.5 X 10⁸ cells per ml). The hemolytic titer is higher when incubation times are prolonged and reaches 50,000 to 100,000 HU/mg after 4 hours at room temperature.

Conjugation of *S*. *aureus* alpha-toxin antigens to other molecules not only reduces the alpha-toxin antigen's hemolytic activity, but also permits the induction of an immune response to the other molecule. Indeed, conjugation of a molecule to an *S*. *aureus* alpha-toxin antigen can improve the molecule's antigenic profile, or increase the strength of an immune response to the molecule. This is particularly true for low molecular weight molecules, such as peptides and oligosaccharides, which cannot, on their own, induce a lasting, powerful immune response. Thus, *S*. *aureus* alpha-toxin antigens function as effective carrier proteins. They are particularly useful carriers for bacterial antigens.

In some embodiments of the invention, therefore, an *S*. *aureus* alpha-toxin antigen is conjugated to another molecule. In one embodiment, the other molecule is another bacterial antigen, such as a bacterial polysaccharide or a bacterial glycoprotein. The bacterial antigen may be an *S*. *aureus* antigen or may be derived from another bacterial species. Exemplary bacterial antigens include *S*. *aureus* Type 5, *S*. *aureus* Type 8, *S*. *aureus* 336, leukocidins, such as Panton-Valentine Leukocidin (PVL) antigens, such as LukS-PV and LukF-PV, gamma-hemolysin subunit antigens such as HlgA, HlgB and HlgC, and other leukocidins such as LukM and LukF'-PV from *S*. *aureus,* LukE and LukD from *S*. *aureus,* LukS-I and LukF-I from *S*. *intermedius, S. epidermis* PS1, *S*. *epidermis* GP1, LTA and MSCRAMM.

Thus, vaccines of the invention may comprise an alpha-toxin antigen-Type 5 conjugate, an alpha-toxin antigen-Type 8 conjugate, an alpha-toxin antigen-Type 336 conjugate, an alpha-toxin antigen-PS1 conjugate, an alpha-toxin-leukocidin conjugate, such as an alpha-toxin-PVL conjugate, an alpha-toxin antigen-GP1 conjugate, an alpha-toxin-LTA conjugate, or an,alpha-toxin-MSCRAMM conjugate. In one embodiment, the other antigen is a protective antigen, *e*.*g*., the antigen induces neutralizing antibodies.

Methods of conjugating an *S*. *aureus* alpha-toxin antigen to another molecule, such as a bacterial antigen, are available in the art. For example, a PS1, Type 5 or Type 8 antigen can be activated by 1-ethyl-3-(3-dimethylaminopropyl) carbodiimide (EDC) to form cysteamine derivatives. Alpha-toxin is modified with N-succinimidyl-3-(-2-pyridyldithio)priopionate (SPDP) and then conjugated to the cysteamine derivative of PS1 via thiol replacement. The resulting conjugates can be separated from the non-conjugated antigen by size exclusion chromatography.

In another embodiment, the *S*. *aureus* alpha-toxin antigen is conjugated to a 336 antigen, for example, by activating the hydroxyl groups on the 336 antigen using cyanogen bromide or 1-cyano-4-dimethylamino-pyridinium tetrafluoroborate, and binding through a linker containing nucleophilic group(s) or without a linker, to the alpha-toxin antigen. The resulting conjugates can then be separated from unconjugated antigen.

In another embodiment, the *S*. *aureus* alpha-toxin antigen is conjugated to a PS1 antigen, for example, by modifying the PS1 with adipic acid dihydrazide (ADH) via an EDC-facilitated reaction to prepare adipic acid hydrazide derivative of PS1 (PS1_{AH}). The *S*. *aureus* alpha-toxin antigen is then succinylated and the succinic derivative of the alpha-toxin antigen is conjugated to PS1_{AH}, a step mediated by EDC.

Other useful conjugation methods also are known in the art, *e*.*g*., periodate oxidation followed with reductive amination, carbodiimide treatment, and combinations of such methods. Such methods can provide direct or indirect (through a linker) covalent binding of molecules to an alpha-toxin carrier. Regardless of the method used to conjugate the molecule to the alpha-toxin carrier, the covalent binding of a molecule to carrier can convert the molecule from a T cell independent antigen to a T cell dependent antigen. As a result, the conjugate would elicit a molecule-specific antibody response in immunized animals, in contrast to no such response upon administration of the molecule alone.

Vaccines of the invention may also comprise a pharmaceutically acceptable carrier. A pharmaceutically acceptable carrier is a material that can be used as a vehicle for the antigen because the material is inert or otherwise medically acceptable, as well as compatible with the active agent, in the context of vaccine administration. In addition to a suitable excipient, a pharmaceutically acceptable carrier can contain conventional vaccine additives like diluents, adjuvants and other immunostimulants, antioxidants, preservatives and solubilizing agents. For example, polysorbate 80 may be added to minimize aggregation and act as a stabilizing agent, and a buffer may be added for pH control.

Methods for making vaccines are generally known in the art. *See, for example,* Di Tommaso et al., Vaccine, 15:1218-24 (1997), and Fattom et al., Infect. and Immun. 58:2367-2374 (1990) and 64:1659-1665 (1996). The vaccines described herein allow for the addition of an adjuvant with relative ease and without distorting the composition. In addition, the vaccines of the present invention may be formulated so as to include a "depot" component to increase retention of the antigenic material at the administration site. By way of example, in addition to an adjuvant (if one is used), alum (aluminum hydroxide or aluminum phosphate), QS-21, dextran sulfate or mineral oil may be added to provide this depot effect.

As described above, vaccines of the invention may comprise one or more bacterial antigens other than an *S*. *aureus* alpha-toxin antigen. The other bacterial antigen may be conjugated to an *S*. *aureus* alpha-toxin antigen, may be co-administered with an *S*. *aureus* alpha-toxin antigen as a separate component of the same composition, or may be administered as part of an entirely separate composition, before, during or after administration of the alpha-toxin antigen. In any case, the other bacterial antigen may be one of those previously described, such as a bacterial polysaccharide or a bacterial glycoprotein, including both *S*. *aureus* antigens and antigens derived from other bacterial species. In one embodiment, the other bacterial antigen is a protective antigen that induces neutralizing antibodies.

Thus, the other bacterial antigen may be the Type 5 and Type 8 antigens described in Fattom et al., Infec. and Immun., 58:2367-2374 (1990), and Fattom et al., Infec. and Immun., 64:1659-1665 (1996). The other bacterial antigen may also be the *S*. *aureus* 336 antigen described in U.S. Patent Nos. 5,770,208; 6,194,161; 6,537,559 or the *Staphylococcal* 336 antigen described in U.S. Patents No. 5,770,208 and No. 6,194,161, or antibodies thereto. Still other *S*. *aureus* antigens are known in the art and are encompassed by the invention. *See, e.g.,* Adams et al., J. Clin. Microbiol., 26:1175-1180 (1988), Rieneck et al., Biochim. Biophys. Acta., 1350:128-132 (1977) and O'Riordan et al., Clin. Microbiol. Rev., 17: 218-34 (2004). For example, Panton-Valentine Leukocidin (PVL) antigen, including its individual subunits LukF-PV and LukS-PV, are encompassed by the invention.

Similarly, the invention embraces *S. epidermidis* antigens. For example, the *S*. *epidermidis* Type II antigen, also referred to a PS1, is disclosed in U.S. Patents No. 5,961,975 and No. 5,866,140. This antigen is an acidic polysaccharide antigen that can be obtained by a process that comprises growing cells of an isolate *of S. epidermidis* that agglutinates antisera to ATCC 55254 (a Type II isolate). The *S. epidermidis* GP1 antigen is described in published U.S. patent application 2005/0118190. GP1 is common to many coagulase-negative strains of *Staphylococcus,* including *Staphylococcus epidermis, Staphylococcus haemolyticus,* and *Staphylococcus hominis.* The antigen can be obtained from the strain of *Staphylococcus epidermis* deposited as ATCC 202176.

Yet another *Staphylococcus* antigen embraced by the present invention is described in WO 00/56357. This antigen comprises amino acids and a N-acetylated hexosamine in an α configuration, contains no O-acetyl groups, and contains no hexose. It specifically binds with antibodies to a *Staphylococcus* strain deposited under ATCC 202176. Amino acid analysis of the antigen shows the presence of serine, alanine, aspartic acid/asparagine, valine, and threonine in molar ratios of approximately 39:25:16:10:7. Amino acids constitute about 32% by weight of the antigen molecule.

Other antigens useful in accordance with the present invention include leukocidins. The class of leukocidins (also referred to as e.g, bicomponent leukotoxins) includes but is not limited to S components, such as LukS-PV, LukM from *S*. *aureus,* HlgA (gamma-hemolysin), HlgC (gamma-hemolysin), LukE from *S*. *aureus,* LukS-I (from *S*. *intermedius),* and F components, such as LukF-PV, LukF'-PV, HlgB (gamma-hemolysin), LukD from *S*. *aureus,* and LukF-I (from *S*. *intermedius).* The present invention encompasses the use of any species of the leukocidin genus, including one or more of the S and F components described herein.

Thus, the invention includes a composition comprising alpha-toxin antigen, one or more additional bacterial antigens, and a pharmaceutically acceptable carrier, where the alpha-toxin antigen and one or more additional bacterial antigens may be provided separately, or where the alpha-toxin antigen is conjugated to one or more additional bacterial antigens.

One embodiment relates to toxin preparations useful, for example, to induce neutralizing antibodies. Exemplary anti-toxin preparations may comprise (i) an *S. aureus* alpha-toxin antigen; (ii) an *S*. *aureus* alpha-toxin antigen and a leukocidin, such as a Panton-Valentine Leukocidin (PVL) antigen; (iii) an *S. aureus* alpha-toxin antigen and one or more PVL antigen subunits, such as LukS-PV or LukF-PV; any combination of (i), (ii), and (iii), and other toxin preparations comprising alpha-toxin antigen. In one specific embodiment, a toxin preparation comprises alpha-toxin and at least one leukocidin antigen, such as at least one PVL subunit or at least one gamma-hemolysin subunit, such as HlgA, HlgB, or HlgC.

Another embodiment relates to opsonic preparations, such as may induce opsonic antibodies. Exemplary opsonic preparations may comprise an alpha-toxin antigen and one or more opsonic antigens, such as *S*. *Aureus* Type 5, Type 8, or 366. An opsonic preparation also may comprise a leukocidin antigen, such as PVL antigen or one or more PVL subunits, such as LukS-PV or LukF-PV. One specific embodiment provides a pentavalent preparation comprising an alpha-toxin antigen, a leukocidin antigen (such a PVL antigen, such as PVL or one or more PVL subunits), Type 5 antigen, Type 8 antigen, and 336 antigen. In another embodiment is a pentavalent combination of antigens that include an rLukS-PV antigen. Another embodiment provides a pentavalent preparation comprising an alpha-toxin antigen, a leukocidin antigen (such as one or more gamma-hemolysin subunit antigens, such as HlgA, HlgB or HlgC), Type 5 antigen, Type 8 antigen, and 336 antigen.

In one embodiment, a preparation comprises both surface antigens and toxin antigens, useful, for example, to prevent *S*. *aureus* infections. Such a composition may comprise surface antigens, such as the Type 5 and/or Type 8 capsular antigens and/or surface polysaccharides such as the 336 antigen, combined with toxin antigens, such as an alpha-toxin antigen (e.g., rALD/H35K) and/or a leukocidin antigen such as a PVL antigen or PVL subunit (e.g., LukS-PV) or gamma-hemolysin subunit antigen. In one embodiment, the composition comprises (i) a Type 5-rEPA conjugate, (ii) a Type 8-rEPA conjugate, (iii) a 336-rEPA conjugate; and (iv) alpha-toxin antigen rALD/H35K. In another embodiment, the composition comprises (i) a Type 5-rEPA conjugate, (ii) a Type 8-rEPA conjugate, (iii) a 336-rEPA conjugate; (iv) alpha-toxin antigen rALD/H35K and (v) rLukS-PV. In another embodiment, the composition comprises (i) a Type 5-rEPA conjugate, (ii) a Type 8-rEPA conjugate, (iii) a 336-rEPA conjugate; (iv) alpha-toxin antigen rALD/H35K and (v) one or more gamma-hemolysin subunit antigens, such as HlgA, HlgB or HlgC.

It has been discovered that some antigens are cross-reactive and cross-neutralizing to infection associated with other antigens. Thus, for example, PVL subunit antigens, such as LukS-PV or LukF-PV, may induce antibodies that neutralize infection associated with another leukocidin, gamma-hemolysin. Conversely, a gamma-hemolysin antigen, such as HlgA, HlgB and/or HlgC, may induce antibodies that neutralize infection associated with PVL. Thus, one aspect of the invention includes a composition comprising one or more PVL subunit antigens that is useful, for example, against gamma-hemolysin infection. Another aspect of the invention includes a composition comprising one or more gamma-hemolysin antigens, such as HlgA, HlgB or HlgC, that is useful, for example, against PVL infection. Thus, the invention includes compositions comprising one type of antigen that are useful against infection associated with a different but cross-reactive antigen.

### Treatment and Prevention of Infections with Vaccine Compositions

The present invention also provides a method of treating or preventing an infection by administering any of the above-described vaccines to a subject in need thereof. A target subject population for the treatment and prevention methods described herein includes mammals, such as humans, who are infected with, or at risk of being infected by, bacterial pathogens, such a *S*. *aureus.* In some embodiments, the infection to be treated or prevented is associated with a methicillin-resistant *S*. *aureus.* In particular embodiments, the methicillin-resistant *S*. *aureus* produces alpha-toxin.

The vaccine may be administered in conjunction with an additional antigen, as described above. Exemplary additional antigens include *S*. *aureus* capsular polysaccharide antigens, such as the Type 5, Type 8, and 336 antigens and other *S*. *aureus* known in the art. Exemplary additional antigens also include *S*. *epidermidis* antigens, such as the PS 1 antigen or the GP1 antigen, and other *Staphylococcus* antigens, such as the antigen described in WO 00/56357. Other exemplary antigens include leukocidins, such as Panton-Valentine Leukocidin (PVL) antigens, such as LukS-PV and LukF-PV, gamma-hemolysin subunit antigens such as HlgA, HlgB and HlgC, and other leukocidins such as LukM and LukF'-PV from *S*. *aureus,* LukE and LukD from *S*. *aureus,* and LukS-I and LukF-I from *S*. *intermedius.* As indicated above, the one or more additional antigens may be administered separately from the *S*. *aureus* alpha-toxin antigen vaccine composition or may be included in the *S*. *aureus* alpha-toxin antigen vaccine composition.

In view of the cross-reactivity and cross-neutralizing activity of some antigens, noted above, the invention includes methods of neutralizing infection associated with one antigen by administering a vaccine comprising a different but cross-reactive antigen. For example, the invention includes methods of neutralizing PVL infection using vaccines comprising gamma-hemolysin antigens such as HlgA, HlgB and/or HlgC, as well as methods of neutralizing gamma-hemolysin infection using vaccines comprising PVL subunit antigens, such as LukF-PV and LukS-PV.

A therapeutically or prophylactically effective amount of the inventive vaccines can be determined by methods that are routine in the art. Skilled artisans will recognize that the amount may vary with the composition of the vaccine, the particular subject's characteristics, the selected route of administration, and the nature of the bacterial infection being treated or prevented. General guidance can be found, for example, in the publications of the International Conference on Harmonization and in REMINGTON'S PHARMACEUTICAL SCIENCES (Mack Publishing Company 1990). A typical vaccine dosage may range from 1 µg - 400 µg of antigen.

The vaccine may be administered with or without an adjuvant. If an adjuvant is used, it is selected so as to avoid adjuvant-induced toxicity. For example, a vaccine according to the present invention may comprise a β-glucan as described in U.S. Patent No. 6,355,625, or a granulocyte colony stimulating factor.

The vaccine may be administered in any desired dosage form, including dosage forms that may be administered to a human intravenously, intramuscularly, or subcutaneously. The vaccine may be administered in a single dose, or in accordance with a multi-dosing protocol. Administration may be by any number of routes, including subcutaneous, intracutaneous, and intravenous. In one embodiment, intramuscular administration is used. The skilled artisan will recognize that the route of administration will vary depending on the bacterial infection to be treated or prevented and the composition of the vaccine.

The vaccine may be administered in conjunction with an anti-infective agent, an antibiotic agent, and/or an antimicrobial agent, in a combination therapy. Exemplary anti-infective agents include, but are not limited to vancomycin and lysostaphin. Exemplary antibiotic agents and antimicrobial agents include, but are not limited to penicillinase-resistant penicillins, cephalosporins and carbapenems, including vancomycin, lysostaphin, penicillin G, ampicillin, oxacillin, nafcillin, cloxacillin, dicloxacillin, cephalothin, cefazolin, cephalexin, cephradine, cefamandole, cefoxitin, imipenem, meropenem, gentamycin, teicoplanin, lincomycin and clindamycin. The dosages of these antibiotics are well known in the art. *See, for example,* MERCK MANUAL OF DIAGNOSIS AND THERAPY, § 13, Ch. 157, 100th Ed. (Beers & Berkow, eds., 2004). The anti-infective, antibiotic and/or antimicrobial agents may be combined prior to administration, or administered concurrently or sequentially with the vaccine composition.

### Antibodies

The present invention further provides compositions comprising antibodies that specifically bind to an *S*. *aureus* alpha-toxin antigen (an "alpha-toxin antibody") and antibodies that specifically bind to another bacterial antigen (a "bacterial antigen antibody"). The *S. aureus* alpha-toxin antigen and other bacterial antigen may be any naturally occurring alpha-toxin or other bacterial antigen, or maybe be any of the antigens described above. The antibodies may be monoclonal antibodies, polyclonal antibodies, antibody fragments or any combination thereof. The antibodies may be formulated with a pharmaceutically acceptable carrier.

The term "antibody," as used herein, refers to a full-length (i.e., naturally occurring or formed by normal immunoglobulin gene fragment recombinatorial processes) immunoglobulin molecule *(e.g.,* an IgG antibody) or an immunologically active (*i*.*e*., specifically binding) portion of an immunoglobulin molecule, including an antibody fragment. "Antibody" and "immunoglobulin" are used synonymously herein. An antibody fragment is a portion of an antibody such as F(ab')₂, F(ab)₂, Fab', Fab, Fv, sFv and the like. Regardless of structure, an antibody fragment binds with the same antigen that is recognized by the full-length antibody, and, in the context of the present invention, specifically binds an *S*. *aureus* alpha-toxin antigen or another bacterial antigen. Methods of making and screening antibody fragments are well-known in the art.

An alpha-toxin antibody or bacterial antigen antibody of the present invention may be prepared by a number of different methods. For example, the antibodies may be obtained from subjects administered an *S*. *aureus* alpha-toxin antigen and/or a bacterial antigen. The antibodies also may be obtained from plasma screened for alpha-toxin antibodies and/or bacterial antigen antibodies, as discussed in more detail below. In some embodiments, the antibodies may be made by recombinant methods. Techniques for making recombinant monoclonal antibodies are well-known in the art. Recombinant polyclonal antibodies can be produced by methods analogous to those described in U.S. Patent Application 2002/0009453 (Haurum et al.), using an *S*. *aureus* alpha-toxin antigen and/or a bacterial antigen as the immunogen(s).

An alpha-toxin antibody or bacterial antigen antibody in accordance with the invention may be a murine, human or humanized antibody. A humanized antibody is a recombinant protein in which the CDRs of an antibody from one species; *e*.*g*., a rodent, rabbit, dog, goat, horse, or chicken antibody (or any other suitable animal antibody), are transferred from the heavy and light variable chains of the rodent antibody into human heavy and light variable domains. The constant domains of the antibody molecule are derived from those of a human antibody. Methods for making humanized antibodies are well known in the art.

The above-described antibodies can be obtained by conventional methods. For example, an alpha-toxin antigen and/or other bacterial antigen can be administered to a subject and the resulting IgGs can be purified from plasma harvested from the subject by standard methodology. The antigens used to obtain antibodies may be any naturally occurring antigen, any of the antigens described above, or any other antigens known in the art. In one embodiment, the *S*. *aureus* alpha-toxin antigen used to obtain alpha-toxin antibody is rendered non-toxic according the teachings above.

### Antibody Compositions

The invention includes antibody compositions suitable for administration, such as compositions comprising an antibody and a pharmaceutically acceptable carrier. The antibody compositions may be formulated for any route of administration, including intravenous, intramuscular, subcutaneous and percutaneous, by methods that are known in the art. In one embodiment, the antibody composition provides a therapeutically or prophylactically effective amount of antibody, i.e., an amount sufficient to achieve a therapeutically or prophylactically beneficial effect. In a further embodiment, the antibody is a protective antibody composition that neutralizes infection and/or provides protection against infection.

In one embodiment, the antibody composition is an IVIG composition. As used herein, "IVIG" refers to an immunoglobulin composition suitable for intravenous administration. IVIG compositions may contain, in addition to immunoglobulin, a pharmaceutically acceptable carrier. The IVIG compositions may be "specific IVIG," meaning that the IVIG contains immunoglobulins that specifically bind to an *S*. *aureus* alpha-toxin antigen and/or other desired bacterial antigen (as described above). The IVIG compositions also may be "hyperimmune specific IVIG." "Hyperimmune specific IVIG" refers to an antibody preparation comprising high titres of alpha-toxin antibodies. A hyperimmune specific IVIG preparation can be prepared from the plasma of a subject that has been challenged with the target *S*. *aureus* alpha-toxin antigen and/or other desired bacterial antigen, or can be obtained by screening plasma of subjects who have not been administered the antigen for high titres of antibody. In either case, the subject may be either a human or animal.

In one embodiment, the specific IVIG composition comprises both an antibody that specifically binds to an *S*. *aureus* alpha-toxin antigen (and that optionally neutralizes the alpha-toxin antigen) and an antibody that specifically binds to another bacterial antigen (and that optionally neutralizes the other bacterial antigen). The antibodies and antigens may be any of those previously described. For example, the other bacterial antigen may be a polysaccharide and may be a glycoprotein, including *S*. *aureus* Type 5, *S*. *aureus* Type 8, *S*. *aureus* 336, *S. epidermidis* PS1, *S*. *epidermidis* GP1, leukocidin components such as PVL (including the individual PVL subunits, LukS-PV and LukF-PV) gamma-hemolysin subunits (HlgA, HlgB, and HlgC), Luk E or LukD from *S*. *aureus,* LukM or LukF'-PV from *S*. *aureus,* a LukF-I a LukS-I subunit from *S*. *intermedius,* lipoteichoic acid (LTA) and microbial surface components recognizing adhesive matrix molecule (MSCRAMM) proteins.

One embodiment relates to anti-toxin preparations. Exemplary anti-toxin preparations may comprise (i) antibodies that specifically bind to an *S*. *aureus* alpha-toxin antigen; (ii) antibodies that specifically bind to an *S*. *aureus* alpha-toxin antigen and antibodies that specifically bind to a leukocidin, such as a Panton-Valentine Leukocidin (PVL) antigen; (iii) antibodies that specifically bind to an *S*. *aureus* alpha-toxin antigen and antibodies that specifically bind to a leukocidin subunit antigen, such as a PVL antigen subunit, such as antibodies that specifically bind to LukS-PV or LukF-PV; any combination of (i), (ii), and (iii), and other anti-toxin preparations comprising antibodies that specifically bind to alpha-toxin antigen. In one specific embodiment, an anti-toxin preparation comprises antibodies that specifically bind to alpha-toxin and antibodies that specifically bind to PVL, LukS-PV or LukF-PV or to another leukocidin such as a gamma-hemolysin subunit, such as HlgA, HlgB, or HlgC.

Another embodiment relates to opsonic antibody preparations, comprising opsonic antibodies. Exemplary opsonic antibody preparations may comprise antibodies that specifically bind to an alpha-toxin antigen and one or more opsonic antibodies, such as antibodies that specifically bind to *S*. *aureus* Type 5, Type 8, or 366. An opsonic antibody preparation also may comprise antibodies that specifically bind to a leukocidin antigen, such as a PVL antigen, or that specifically bind to one or more PVL subunits, such as LukS-PV or LukF-PV, or to a gamma-hemolysin subunit, such as HlgA, HlgB, or HlgC. One specific embodiment provides a pentavalent preparation comprising antibodies that specifically bind to an alpha-toxin antigen, antibodies that specifically bind to a leukocidin antigen such as a PVL antigen (such as PVL or one or more PVL subunits) or a gamma-hemolysin subunit (such as HlgA, HlgB, or HlgC), antibodies that specifically bind to Type 5 antigen, antibodies that specifically bind to Type 8 antigen, and antibodies that specifically bind to 336 antigen.

Thus, some embodiments provide compositions comprising monoclonal and/or polyclonal antibodies which are neutralizing (such as anti-alpha-toxin antibodies) and/or opsonizing (such antibodies against capsular or surface antigens). One composition comprises monoclonal and/or polyclonal antibodies that specifically bind to Type 5 antigen, antibodies that specifically bind to Type 8 antigen, antibodies that specifically bind to 336 antigen, and antibodies that specifically bind to alpha-toxin antigen rALD/H35K. Another composition comprises monoclonal and/or polyclonal antibodies that specifically bind to Type 5 antigen, antibodies that specifically bind to Type 8 antigen, antibodies that specifically bind to 336 antigen, antibodies that specifically bind to alpha-toxin antigen and antibodies that specifically bind to rLukS-PV. Another composition comprises monoclonal and/or polyclonal antibodies that specifically bind to Type 5 antigen, antibodies that specifically bind to Type 8 antigen, antibodies that specifically bind to 336 antigen, antibodies that specifically bind to alpha-toxin antigen and antibodies that specifically bind to a gamma-hemolysin subunit, such as HlgA, HlgB, or HlgC.

Another embodiment relates to cross-reactive, cross-neutralizing antibody compositions. For example, the invention includes compositions comprising antibodies that are specific to one antigen and that are cross-reactive and cross-neutralizing to another antigen. For example, the invention includes compositions comprising antibodies specific to gamma-hemolysin antigens, such as HlgA, HIgB and/or HlgC, that are useful, for example, against PVL infection, as well as compositions comprising antibodies specific to PVL subunit antigens, such as LukF-PV and LukS-PV, that are useful against gamma-hemolysin infection.

As noted above, the invention provides antibody compositions that provide a therapeutically or prophylactically effective amount of antibody, i.e., an amount sufficient to achieve a therapeutically or prophylactically beneficial effect. In a further embodiment, the antibody is a protective antibody composition that neutralizes infection and/or provides protection against infection. Such protective compositions may include a protective amount of an alpha-toxin antibody and a protective amount of antibody against another bacterial antigen. Alternatively, a protective antibody composition may comprise a sub-optimal amount of anti-alpha-toxin antibody and a sub-optimal amount of antibody against another bacterial antigen. As used herein, "sub-optimal" amount means an amount that is not protective on its own, i.e., an amount that is not effective, on its own, to neutralize infection or to provide protection against infection. These compositions, while comprising amounts of antibody that are not effective on their own, nevertheless neutralize infection and/or provide protection against infection by the synergistic activity of the combination of antibodies. In one specific embodiment, the composition comprises a sub-optimal amount of anti-alpha-toxin antibody and a sub-optimal amount of *S*. *aureus* Type 5 antibody. In another specific embodiment, the composition comprises a sub-optimal amount of anti-alpha-toxin antibody and a sub-optimal amount of *S*. *aureus* Type 8 antibody.

### Methods of Making IVIG Compositions

The present invention also provides methods of making IVIG compositions, including specific IVIG compositions and hyperimmune IVIG compositions. Any of the antigen compositions mentioned above can be used to make IVIG compositions. In one embodiment, an IVIG composition is prepared by administering an *S*. *aureus* alpha-toxin antigen and another bacterial antigen to a subject, then harvesting plasma from the subject and purifying immunoglobulin from the plasma. The *S. aureus* alpha-toxin antigen and other bacterial antigen may be any of those described above, including wildtype antigens, and protective antigens that induce neutralizing antibodies, and may be formulated in any of the above-described vaccines. Thus, the other bacterial antigen may be a polysaccharide and may be a glycoprotein, and in one embodiment is selected from S. *aureus* Type 5, *S*. *aureus* Type 8, *S*. *aureus* 336, *S. epidermidis* PS1, *S*. *epidermidis* GP1, leukocidins, e.g., leukocidin components such as Panton-Valentine Leukocidin (PVL) antigens, such as LukS-PV and LukF-PV, gamma-hemolysin subunit antigens such as HlgA, HlgB and HlgC, and other leukocidins such as LukM amd :ukF'-PV from *S*. *aureus,* LukE and LukD from *S*. *aureus,* LukS-I and LukF-I from *S*. *intermedius,* lipoteichoic acid (LTA) and microbial surface components recognizing adhesive matrix molecule (MSCRAMM) proteins. The bacterial antigen may be conjugated to the *S*. *aureus* alpha-toxin antigen. In one embodiment, the *S*. *aureus* alpha-toxin antigen contains at least two alterations, relative to wild-type *S*. *aureus* alpha-toxin, that reduce its toxicity, as described above.

The subject that is challenged, or administered, the antigen(s), such as the *S*. *aureus* alpha-toxin antigen and other bacterial antigen, may be a human or may be another animal, such as a mouse, a rabbit, a rat, a chicken, a horse, a dog, a non-human primate, or any other suitable animal. Antibodies that specifically bind the antigen(s) may be obtained from the animal's plasma by conventional plasma-fractionation methodology.

In another embodiment, IVIG compositions are prepared by screening a subject that has not been administered the antigen(s), such as a subject that has not been administered an *S*. *aureus* alpha-toxin antigen and another bacterial antigen (*i*.*e*., an unstimulated subject), then harvesting plasma from the subject and immunoglobulin from the plasma. In this embodiment, plasma from unstimulated subjects is screened for high titers of antibodies that specifically bind to the antigen(s), such as the *S*. *aureus* alpha-toxin antigens and other bacterial antigen(s). The antigens may be any of those described above. For example, the other bacterial antigen(s) may be a polysaccharide and may be a glycoprotein, and may be selected from *S*. *aureus* Type 5, *S*. *aureus* Type 8, *S*. *aureus* 336, *S. epidermidis* PS1, *S*. *epidermidis* GP1, a leukocidin such as PVL (including the individual PVL subunits, LukS-PV and LukF-PV) or and gamma-hemolysin subunit (HlgA, HlgB, or HlgC), LukE or LukD from *S*. *aureus,* LukM or LukF'-PV from *S. aureus,* a LukF-I or LukS-I subunit from *S*. *intermedius,* lipoteichoic acid (LTA) and microbial surface components recognizing adhesive matrix molecule (MSCRAMM) proteins. In accordance with one embodiment, plasma is screened for alpha-toxin antibody and/or other bacterial antibody titers that are 2-fold or more, 3-fold or more, 4-fold or more, or 5-fold or more higher than the levels typically found in standard IVIG preparations.

Again, the subject to be screened may be a human or may be another animal, such as a mouse, a rabbit, a rat or a non-human primate. Immunoglobulin may be obtained from the animal's plasma by conventional plasma-fractionation methodology.

### Treatment and Prevention of Infections with Antibody Compositions

The present invention also provides a method of treating or preventing infection by administering the above-described antibody compositions, such as the above-described IVIG compositions, to a subject in need thereof. A target patient population for the treatment and prevention of infection includes mammals, such as humans, who are infected with or at risk of being infected by bacterial pathogens. In one embodiment, the infection to be treated or prevented is an *S*. *aureus* infection, including an infection of methicillin-resistant *S*. *aureus* or *S*. *aureus* that produces alpha-toxin, or an *S*. *epidermidis* infection.

In accordance with one embodiment, the invention provides a method for treating or preventing an *S*. *aureus* infection using compositions comprising an *S*. *aureus* alpha-toxin antibody, an antibody that specifically binds to another *S*. *aureus* antigen, and a pharmaceutically acceptable carrier. The *S*. *aureus* alpha-toxin antibody and the antibody that binds to another *S*. *aureus* antigen may be any of those described above. In one embodiment, the antibody composition is an IVIG composition or a hyperimmune specific IVIG composition. In another embodiment, the antibodies are recombinant or humanized antibodies. In yet another embodiment, the antibodies are monoclonal antibodies.

In view of the cross-reactivity and cross-neutralizing activity of some antigens noted above, the invention includes methods of neutralizing infection associated with one antigen by administering an antibody composition (including an IVIG composition) comprising antibody specific to a different antigen that is cross-reactive and cross-neutralizing to the first antigen. For example, the invention includes methods of neutralizing PVL infection using antibody compositions or IVIG comprising antibody specific to gamma-hemolysin antigens, such as HlgA, HlgB and/or HlgC, as well as methods of neutralizing gamma-hemolysin infection using antibody compositions or IVIG comprising antibody specific to PVL subunit antigens, such as LukF-PV and LukS-PV.

A therapeutically or prophylactically effective amount of the antibody compositions can be determined by methods that are routine in the art. Skilled artisans will recognize that the amount may vary according to the particular antibodies within the composition, the concentration of antibodies in the composition, the frequency of administration, the severity of infection to be treated or prevented, and subject details, such as age, weight and immune condition. In some embodiments, the dosage will be at least 50 mg IVIG composition per kilogram of body weight (mg/kg), including at least 100 mg/kg, at least 150 mg/kg, at least 200 mg/kg, at least 250 mg/kg, at least 500 mg/kg, at least 750 mg/kg and at least 1000 mg/kg. Dosages for monoclonal antibody compositions typically may be lower, such as 1/10 of the dosage of an IVIG composition, such as at least about 5 mg/kg, at least about 10 mg/kg, at least about 15 mg/kg, at least about 20 mg/kg, or at least about 25 mg/kg. The route of administration may be any of those appropriate for a passive vaccine. Thus, intravenous, subcutaneous, intramuscular, intraperitoneal and other routes of administration are envisioned. As noted above, a therapeutically or prophylactically effective amount of antibody is an amount sufficient to achieve a therapeutically or prophylactically beneficial effect. A protective antibody composition may neutralize and/or prevent infection. A protective antibody composition may comprise amounts of anti-alpha-toxin antibody and/or antibody against another bacterial antigen that are not protective on their own, but which, in combination, yield a protective antibody composition.

The antibody composition may be administered in conjunction with an anti-infective agent, an antibiotic agent, and/or an antimicrobial agent, in a combination therapy. Exemplary anti-infective agents include, but are not limited to vancomycin and lysostaphin. Exemplary antibiotic agents and antimicrobial agents include, but are not limited to penicillinase-resistant penicillins, cephalosporins and carbapenems, including vancomycin, lysostaphin, penicillin G, ampicillin, oxacillin, nafcillin, cloxacillin, dicloxacillin, cephalothin, cefazolin, cephalexin, cephradine, cefamandole, cefoxitin, imipenem, meropenem, gentamycin, teicoplanin, lincomycin and clindamycin. The dosages of these antibiotics are well known in the art. *See, for example,* MERCK MANUAL OF DIAGNOSIS AND THERAPY, § 13, Ch. 157, 100th Ed. (Beers & Berkow, eds., 2004). The anti-infective, antibiotic and/or antimicrobial agents may be combined prior to administration, or administered concurrently or sequentially with the IVIG composition.

In some embodiments, relatively few doses of antibody composition are administered, such as one or two doses, and conventional antibiotic therapy is employed, which generally involves multiple doses over a period of days or weeks. Thus, the antibiotics can be taken one, two or three or more times daily for a period of time, such as for at least 5 days, 10 days or even 14 or more days, while the antibody composition is usually administered only once or twice. However, the different dosages, timing of dosages and relative amounts of antibody composition and antibiotics can be selected and adjusted by one of ordinary skill in the art.

The following examples are illustrative only, rather than limiting, and provide a more complete understanding of the invention.

### Example 1

This example demonstrates the cloning and expression of a recombinant alpha-toxin mutant ALD/H35K (rALD/H35K) in *Escherichia coli.* rALD/H35K contains a deletion of the amino latch (ALD) and a point mutation at amino acid position 35, histidine to lysine (H35K).

An expression construct for recombinant alpha-toxin mutant protein rALD/H35K without a histidine-6-tag was prepared as follows. The ALD/H35K gene was PCR amplified from a previously prepared histidine-tagged construct, pTrcHis-ALD/H35K. Primers were designed to remove the histidine tag and incorporate Ncol and BamHI restriction sites at the amino and carboxy termini, respectively. After amplification and restriction digestion, the ALD/H35K gene was ligated into the Invitrogen pTrcHisB vector at the Ncol and BamHI restriction sites. By using the ATG of the Ncol restriction site for initiation of translation, the vector-encoded histidine tag and enterokinase cleavage site were removed. The result was the expression of the protein without additional N-terminal amino acids.

A double restriction digestion of pTrcHis-B was performed using Nco I and BamHI. A PCR reaction was then used to create the double mutant, ALD/H35K, without the His6-Tag. Primers for the PCR reaction were ALD-F (5'-GGCAGCATGCCATGGCAAATACTACAGTAAAAAC-3') (SEQ ID NO: 1) and AGO-2 (5'-GGAATTCGTGGATCCTTAATTTGTCATTTCTTC-3') (SEQ ID NO: 2). After PCR, agarose gel electrophoresis was performed. After the gel was analyzed and photographed, the gel was placed onto a UV transilluminator and the vector and insert (PCR products) were excised. The vector and insert were extracted from the agarose slices using a matrix gel extraction system. Ammonium acetate/ethanol precipitation of the PCR product were performed, followed by a double restriction digestion of the gel extracted insert using Nco I and BamHI. Silica resin purification of the digested insert was then performed.

The vector and insert were ligated according to instructions on the Genechoice™ Rapid Ligation Kit. The ligation products were then transformed into GC10 competent high efficiency cells, which were grown on transformation plates. Screening by "colony PCR" was then performed. Overnight cultures of colonies producing the correct sized amplicons (∼800 bp) were grown. Bead stocks and minipreps of potential clones were prepared. A restriction enzyme digestion analysis of the minipreps was performed and the minipreps were quantitated for sequencing purposes. Sequencing was performed using four primers: pTrcHis-Forward (5'-GAGGTATATATTAATGTATCG-3') (SEQ ID NO: 3), Forward-1 (5'-GGTACCATTGCTGG-3') (SEQ ID NO: 4), Forward-2 (5'-CGATTGGTCATACACTG-3') (SEQ ID NO: 5), and Forward-3 (5'-CCAGACTTCGCTAC-3') (SEQ ID NO: 6). Sequencing verified the correct DNA sequence of the insert.

Cultures also were grown from the bead stocks and protein expression was analyzed by SDS-PAGE after cell lysis. Soluble over-expression of the rALD/H35K alpha-toxin mutant was confirmed.

### Example 2

This example demonstrates the construction of alpha-toxin mutants that lack the toxic or hemolytic activity of wild type alpha-toxin from *S*. *aureus.* Mutants His35 substitution/deletion, Amino Latch Deletion (ALD) and Stem Deletion (SDD) were constructed to disrupt the heptameric pore. These regions are believed to play critical role in pore formation. The mutants were made as recombinant proteins, and were constructed by PCR cloning techniques. The mutants were then IPTG induced to express the protein, which were evaluated for their toxicity/hemolytic activity.

Genomic DNA was purified from *S*. *aureus* Wood 46 strain using Wizard™ genomic DNA purification kit from Promega. PCR was performed using the primer combinations set forth in Tables 1 & 2 below.

**Table 1**

| **Primer Name** | **SEQ ID NO:** | **Sequence** |
|---|---|---|
| KT01 | 7 | 5' GCATGCCATGGCAGATTCTGATATTAAT 3' |
| KT02 | 8 | 5' CGTGGATCCTTAATTTGTCATTTCTTC 3' |
| KT03 | 9 | 5'GAAAATGGCATGAAAAAAGTATTTTATAG 3' |
| KT04 | 10 | 5'CTATAAAATACTTTTTTCATGCCATTTTC 3' |
| AG01 | 11 | 5'GGCAGCATGCCATGGCAGATTCTGATATTAAT 3' |
| AG02 | 12 | 5'GGAATTCGTGGATCCTTAATTTGTCATTTCTTC 3' |
| AG03 | 13 | 5'GAAAATGGCATGTTGAAAAAAGTATTTTATAG 3' |
| AG04 | 14 | 5'CTATAAAATACTTTTTTCAACATGCCATTTTC 3' |
| AG05 | 15 | 5'GAAAATGGCATGGCAAAAAAAGTATTTTATAG 3' |
| AG06 | 16 | 5'CTATAAAATACTTTTTTTGCCATGCCATTTTC 3' |
| H48L-F | 17 | 5'CGATGATAAAAATCTGAATAAAAAACTGC 3' |
| H48L-R | 18 | 5'GCAGTTTTTTATTCAGATTTTTATCATCG 3' |
| H48E-F | 19 | 5'CGATGATAAAAATGAAAATAAAAAACTGC 3' |
| H48E-R | 20 | 5'GCAGTTTTTTATTTTCATTTTTATCATGC 3' |
| H35K-F | 21 | 5'GAAAATGGCATGAAAAAAAAAGTATTTTATAG |
| H35K-R | 22 | 5'CTATAAAATACTTTTTTTTTCATGCCATTTTG 3' |
| H35R-F | 23 | 5'GAAAATGGCATGAGAAAAAAAGTATTTTATAG 3' |
| H35R-R | 24 | 5'CTATAAAATACTTTTTTTCTCATGCCATTTTG 3' |
| ALD-F | 25 | 5'GGCAGCATGCCATGGCAAATACTACAGTAAAAAC 3' |
| CTH-R | 26 | 5’GGAATTCGTGGATCCTTAGTGATGGTGATGGTGATGA TTTGTCATTTCTTC 3’ |
| SDD-F | 27 | CCAAGAAATTCGATTGATACAAAAGTTCAACCTGATTT CAAAAC 3' |
| SDD-R | 28 | GTTTTGAAATCAGGTTGAACTTTTGTATCAATCGAATTT CTTGG 3' |

**Table 2**

| **Mutant** | **C-terminal His-Tag** | **Primary PCR** | | **Secondary PCR** | | **Annealing Temp** |
|---|---|---|---|---|---|---|
| | | **Template** | **Primers** | **Template** | **Primers** | |
| **wild-type** | No | genomic DNA | AG01 & AG02 | - | - | 50 |
| **H35 del** | No | genomic DNA | AG01 & KT04 | Primary PCR | AG01 & AG02 | 50 |
| | | | KT03 & AG02 | fragments(PPF) | | |
| **H35A** | No | genomic DNA | AG01 & AG06 | PPF | AG01 & AG02 | 50 |
| | | | AG05 & AG02 | | | |
| **H35L** | No | genomic DNA | AG01 & AG04 | PPF | AG01 & AG02 | 50 |
| | | | AG03 & AG02 | | | |
| **ALD** | yes | genomic DNA | ALD-F & CTH-R | - | - | 42 |
| **H35K** | yes | genomic DNA | AG01 & H35K-R | PPF | AG01 & CTH-R | 42 |
| | | | H35K-F & CTH-R | | | |
| **H35R** | yes | genomic DNA | AG01 & H35R-R | PPF | AG01 & CTH-R | 42 |
| | | | H35R-F & CTH-R | | | |
| **H48E** | yes | genomic DNA | AG01 & H48E-R | PPF | AG01 & CTH-R | 42 |
| | | | H48E-F & CTH-R | | | |
| **H48L** | yes | genomic DNA | AG01 & H48L-R | PPF | AG01 & CTH-R | 42 |
| | | | H48L-F & CTH-R | | | |
| **SDD** | yes | genomic DNA | AG01 & SDD-R | PPF | AG01 & CTH-R | 42 |
| | | | SDD-F & CTH-R | | | |
| **H35R-H48E** | yes | H35R | AG01 & H48E-R | PPF | AG01 & CTH-R | 43 |
| | | | H48E-F & CTH-R | | | |
| **H35K-H48E** | yes | H35K | AG01 & H48E-R | PPF | AG01 & CTH-R | 43 |
| | | | H48E-F & CTH-R | | | |

Double restriction digestion of the PCR amplified DNA fragments and pTrcHisB vector DNA were then performed and followed by ammonium acetate and ethanol precipitation of the digested DNA. Restriction digested and ethanol precipitated insert and vector DNA were ligated, and competent E. coli cells were transformed with the ligated DNA and grown on agar plates. Colonies were then picked and plasmid preps were made. The plasmids were digested with BamHI and Nco I enzymes and run on agarose gels to screen for recombinants. Bead stocks were made from the recombinants and sequenced. Sequencing results were matched with the sequence of wild-type alpha-toxin and the presence of the desired mutations was confirmed.

IPTG induction and expression of the mutants also was performed. The mutants were variously expressed in soluble and insoluble forms. The expression was confirmed by SDS-PAGE.

### Example 3

This example demonstrates the purification and characterization of rALD/H35K alpha toxoid without a his-tag.

Cells containing an expression plasmid and induced for the expression of rALD/H35K alpha toxoid were lysed with lysozyme. The membranes were then solubilized with deoxycholic acid (DOC). Viscosity of the cell lysate was then reduced by sonication, followed by a digestion of DNA/RNA with DNase and RNase enzymes. Cell debris was removed by centrifugation, and the supernatant containing the alpha toxoid was decanted for further processing. Chromatography was performed on the supernatant using a column packed with Toyopearl™ Phenyl 650M resin. The Phenyl 650 column fractions were analyzed by SDS-PAGE using a Coomassie staining method and pooled fractions, selected for purity and quantity of alpha toxoid, were subjected to diafiltration. Further chromatography was performed using a column packed with Amersham Cibacron Blue Fast Flow™ resin. The column fractions were again analyzed by SDS-PAGE and pooled fractions, selected for purity and quantity of alpha toxoid, were again subjected to diafiltration. Further chromatography was performed using a column packed with ceramic hydroxyapatite (CHT). Fractions from the CHT column were analyzed by SDS-PAGE, and select fractions were pooled for their purity and quantity of the alpha toxoid. The entire purification process is outlined below.

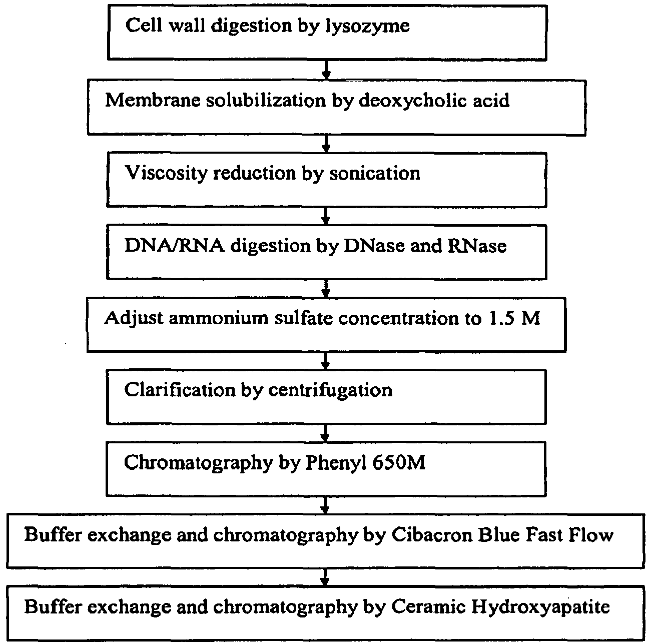

Protein content, purity and molecular weight were confirmed by BCA, SDS-PAGE and size exclusion chromatography. Western blot and N-terminal sequencing confirmed the identity of the rALD/H35K alpha toxoid. Sandwich ELISA showed that the purification process yielded 12% recovery of the rALD/H35K alpha toxoid.

A standard hemolytic assay was performed on the rALD/H35K alpha toxoid, and showed that the toxoid had no hemolytic activity.

### Example 4

This example demonstrates the purification and characterization of recombinant alpha-toxin for use as a carrier protein for making PS-protein conjugate. An H35K/ALD (rALD/H35K) double mutant was constructed and over expressed in *E*. *coli.* The mutant was purified using a Ni-NTA (nickel-charge) affinity column first, then further purified by using a ceramic hydroxyapatite column. The antigenicity and toxicity of the purified alpha-toxin were evaluated by immunodiffusion and hemolytic activity. The *E*. *coli* cells were lysed using B-PER with Benzonase and PMSF. Centrifugation was performed and the supernatant was collected. Chromatography was performed using an Ni-NTA column and collected fractions were analyzed by SDS-PAGE. The alpha-toxin fractions were pooled and analyzed for total protein by BCA protein assay. The Ni-NTA purified alpha-toxin was further purified by chromatography using an HTP column and collected fractions were again analyzed by SDS-PAGE. The alpha-toxin containing fractions were then pooled, concentrated and analyzed. The purification process is outlined below.

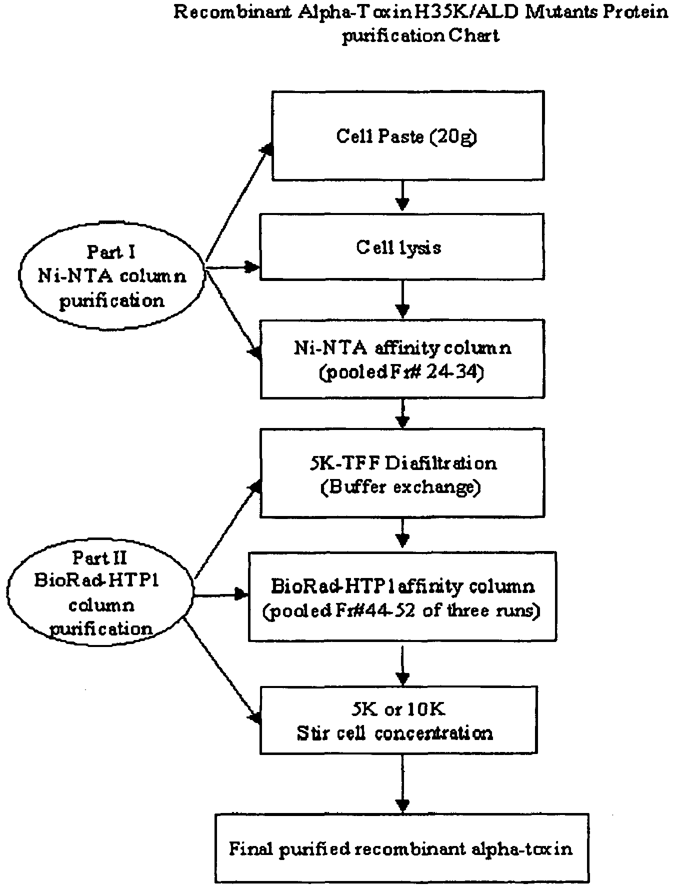

The identity and antigenicity of the purified alpha-toxin mutant were tested by immunodiffusion.

A standard hemolytic assay also was performed on the alpha-toxin mutant, and showed that the mutant had no detectable hemolytic activity.

### Example 5

This example demonstrates that a synergistic passive protection against a high alpha-toxin producing *S*. *aureus* isolate can be achieved by administration of AltaStaph in combination with α-toxin specific antibodies derived against recombinant ALD/H35K α-toxoid mutant.

AltaStaph™ (Nabi™ Biopharmaceuticals, Rockville, Maryland) contains high levels of antibodies to the capsular polysaccharide Type 5 and Type 8 antigens from *S*. *aureus.* AltaStaph™ is produced by immunizing healthy human volunteers with StaphVAX^{®} (Nabi^{®} Biopharmaceuticals, Rockville, Maryland), which comprises capsular polysaccharide *S*. *aureus* Type 5 and Type 8 antigens. As presently produced, Altastaph™ is a sterile, injectable 5% solution of human plasma protein at pH 6.2 in 0.075 sodium chloride, 0.15 M glycine and 0.01% polysorbate 80. Each 1 mL of solution contains 50 mg protein, of which greater than 96% is IgG immunoglobulin. IgA and IgM classes are present at concentrations of ≤ 1.0 g/L.

Eighty female BALB/c mice were randomized into clean cages and quarantined for 6 days prior to study initiation.

Twenty-four hours prior to bacterial challenge, 10 mice per group were administered antibody doses into intra-peritoneal cavity. Group designation for individual antibody treatment is described in Table 3.

**Table 3**

| **(study group designation based on antibody treatment)** | | |
|---|---|---|
| **Group** | **Group Size** | **Immunization Treatment** |
| 1 | 10 | 200 *µ*g T5CP IgG AltaStaph + *α*-toxoid (rALD/H35K) rabbit IgG (4 mg total IgG) |
| 2 | 10 | 200 *µ*g T5CP IgG AltaStaph + *α*-toxoid, (rALD/H35K) rabbit IgG (2 mg total IgG) |
| 3 | 10 | 200 *µ*g T5CP IgG AltaStaph + *α*-toxoid (rALD/H35K) rabbit IgG (1 mg total IgG) |
| 4 | 10 | *α*-toxoid (rALD/H35K) rabbit IgG (4 mg total IgG) |
| 5 | 10 | Non-immune rabbit IgG (4 mg total IgG) |
| 6 | 10 | 200 *µ*g T5CP IgG AltaStaph™ (Total IgG of 3.87 mg) |
| 7 | 10 | MEP IGIV (Total IgG of 3.87 mg) |
| 8 | 10 | PBS (500 *µ*L volume per dose) |

*S. aureus* Type 5 Isolate 328, a high αtoxin producing isolate, was grown overnight for ∼20 hours in 10 mL of Columbia Mg/CaCl₂ media at 37°C with 200 rpm constant shaking. Next day, bacteria were suspended in PBS to an O.D. of 0.1 at 540 nm. This O.D. gave a concentration of ∼2 x10⁷CFU/ml that was than serially adjusted to ∼2 x10⁵ CFU/ml at total volume of 25 mL. The diluted bacterial suspension was placed on ice in preparation for bacterial challenge in combination with freshly prepared hog mucin.

5 grams of hog mucin powder was solubilized in 50 ml phosphate buffered saline (PBS) at room temperature for 5-10 minutes with constant stir. After mixing, suspension was autoclaved for 10 minutes at unwrapped cycle, suspension was ice cooled and transferred to animal facility in ice filled container.

At the time of injection, bacterial suspension was suspended in equal volumes of 10% hog mucin, filled into 3ml syringes, and 500µL injected into mouse peritoneal cavity using 25 G ^{5/8} needles fitted syringes. The calculated actual challenge dose was at 5.81 x10⁴ CFU per 500 µL of challenge. Post-challenge morbidity and mortality per individual group were recorded at 16, 24, 41, 48, 65, 168 hours. The study was terminated on 5^{th} day of post-challenge.

The survival data per individual treated group is outlined in Table 4, below. Mice that were administered 200 µg T5CP specific IgG (AltaStaph™ IGIV) supplemented with 4 mg of α-Toxoid (rALD/H35 double mutant) derived total rabbit IgG showed 100 % protection. The level of protection declined in mice that were immunized with AltaStaph supplemented with either 2 mg or 1 mg toxoid IgG. The survival rate for 2 mg total IgG dose was 90 % while for 1 mg dose was 60 % after five days of challenge. In contrast, non-supplemented AltaStaph had thirty percent survival, while no protection observed with toxoid IgG, MEP IGIV.

**Table 4**

| AltaStaph IGIV + *α*-Toxoid (rALD/H35K) IgG Synergistic passive protection against highly virulent *α*-toxin secreting *Staphylococcus aureus* | | | | | | | | |
|---|---|---|---|---|---|---|---|---|
| **Grp** | **Immunoglobulin Administration (IP) (Day -1 of challenge)** | ***S. aureus,* Isolate 328 Challenge** | **Post-Challenge Survival** | | | | | |
| | | | **(Hours Post-Challenge)** | | | | | |
| | | | **16** | **24** | **41** | **48** | **168** | **Surviv.** |
| 1 | 200 *µ*g T5CP IgG + 4 mg *α*-Toxoid IgG* | 5 x 10⁴ CFU In 5% Mucin | 10/10 | 10/10 | 10/ 10 | 10/10 | 10/10 | 100% |
| 2 | 200 *µ*g T5CP IgG + 2 mg *α* -Toxoid IgG* | | 10/10 | 10/10 | 9/10 | 9/10 | 9/10 | 90% |
| 3 | 200 *µ*g T5CP IgG + 1 mg *α*-Toxoid IgG* | | 8/10 | 8/10 | 6/10 | 6/10 | 6/10 | 60% |
| 5 | 4 mg *α*-Toxoid IgG* | | 10/10 | 0/10 | 0/10 | 0/10 | 0/10 | 0 % |
| 6 | 4 mg Normal rabbit IgG* | | 10/10 | 0/10 | 0/10 | 0/10 | 0/10 | 0 % |
| 7 | 200 *µ*g T5CP IgG | | 4/10 | 3/10 | 3/10 | 3/10 | 3/10 | 30 % |
| 8 | MEP IgG | | 2/10 | 0/10 | 0/10 | 0/10 | 0/10 | 0 % |
| 9 | PBS | | 0/10 | 0/10 | 0/10 | 0/10 | 0/10 | 0 % |

| | | | | | | | | |
|---|---|---|---|---|---|---|---|---|
| * Dose of total IgG. | | | | | | | | |

Individually administered 200 µg capsule-specific IgG of AltaStaph™ IGIV and rabbit IgG derived against α-toxoid (rALD/H35 Mutant) were not protective against highly hemolytic α-toxin secreting *S*. *aureus* lethal challenge. However, combinations of 200 µg T5CP AltaStaphTM human IGIV with α-toxoid antibodies are 100 % protective against hemolytic *S*. *aureus* lethal challenge. The presence of toxin neutralizing antibodies in AltaStaph IGIV provides additional protective efficacy against highly virulent α-toxin secreting *S*. *aureus* isolates.

### Example 6. Generation of Alpha-Toxin rALD/H35K Clone

Genomic DNA was isolated from *S*. *aureus* strain ATCC #10832, Wood 46, a prototype strain that produces alpha-toxin (alpha-hemolysin) obtained from the American Type Culture Collection (ATCC), according to a modified Promega protocol as described using Wizard Genomic DNA purification kit. Oligonucleotide primers were designed to create a H35K point mutation and an amino latch deletion (ALD, ΔA1-N17). The forward primers were designed to eliminate the putative signal peptides and incorporate an NcoI site. The ATG of the Ncol site was designed to serve as the start codon for translation, eliminating the addition of vector encoded N-terminal amino acids. The reverse primers were designed to incorporate a BamHI site immediately downstream of the stop codon. Using the *hla* gene of the Wood 46 genomic DNA as a template, PCR was used to create single mutants, H35K and ALD, and the double mutant, ALD/H35K (with and without His6 tags).

### Primers used:

ALD: forward primer with NcoI site, start codon follows gene sequence for ALD:
5' GGCAGCATGCCATGGCAAATACTACAGTAAAAAC 3'
AGO-2: reverse primer encoding BamHI site and stop codon:
5' GGAATTCGTGGATCCTTAATTTGTCATTTCTTC 3'
H35K-F: forward primer encoding the H35K mutation:
5' GAAAATGGCATGAAAAAAAAAGTATTTTATAG
H35K-R: reverse primer encoding the H35K mutation:
5'CTATAAAATACTTTTTTTTTCATGCCATTTTG 3'
CTH-R: C-terminal primer encoding His6 tag stop codon and BamHI site:
5'GGAATTCGTGGATCCTTAGTGATGGTGATGGTGATGATTTGTCATTTCTTC 3'
AG01: N-terminal primer encoding Ncol site, start codon, followed by the *hla* gene:
5'GGCAGCATGCCATGGCAGATTCTGATATTAAT 3'

The PCR products were cloned into pTrcHisB or using the Ncol and BamHI sites as the described procedure by the manufacturer (Invitrogen). In addition, the NcoI-BamHI insert containing the *hla ALD*/*H35K* gene was subsequently subcloned into pET28 (Novagen).

The resulting constructs were transformed into *E*. *coli* GC10 cells using the manufacturer's protocol (Gene Choice). Sequencing was performed using ABI PRISM Dye Terminator Cycle Sequencing. All clones with the correct sequence were transformed into *E*. *coli* GC10 or *E. coli* BL21 (DE3) pLysS as for expression.

### Example 7. Expression and Purification of rALD/H35K

In shake flasks the *E*. *coli* strain GC10 or BL21 (DE3) pLysS containing the rALD/H35K plasmid was cultured in selective medium at 37°C until mid-log phase and induced using final concentration of 1 mM IPTG for 2-3 hours. The cells were harvested by centrifugation. Analysis of the shake-flask cultures by SDS-PAGE and Western blot analysis showed a band with an apparent molecular weight of 32 KDa that was not evident prior to induction. This molecular weight that was observed was consistent with the mutations present, while wild type recombinant alpha-toxin has an apparent molecular weigh of 34 KDa.

The pelleted cells were resuspended in 20 mM Tris-HCl, 50 mM NaCl, pH 8 and treated with 2 mg/g paste of lysozyme at room temperature for 20 min, followed by membrane disruption with 0.25% (w/v) deoxycholic acid and sonication with a Misonix sonicator. The disrupted cell suspension was mixed with equal volume of 2.25 M of (NH₄)₂SO₄, 20 mM Na₂HPO₄, pH 7.0 buffer. The supernatant of cell lysate was collected by centrifugation.

The soluble protein was chromatographed on a Toyopearl ® Phenyl-650M. The bound rALD/H35K mutant was eluted using a linear gradient of 1.5 to 0 M of(NH₄)₂SO₄ and 0 to 20% glycerol in 20 mM Na₂HPO₄, pH 7.0 buffer. The rALD/H35K containing fractions were pooled and diafiltered against 20 mM Tris, 100 mM NaCl, 5% glycerol, pH 7.0. The resulting diafiltered fractions were applied on a Blue Sepharose 6 FF column and eluted with a linear gradient of 0.1 to 2.5 M of NaCl in 20 mM Tris, 5% glycerol, pH 7.0 buffer. The rALD/H35K containing fractions were pooled and diafiltered against 20 mM Na₂HPO₄, 100 mM NaCl, 5% glycerol, pH 6.8 buffer. The retentate was then chromatographed on a Ceramic Hydroxyapatite Type I column using a linear gradient of 100 to750 mM NaCl in 20 mM Na₂HPO₄, 5% glycerol, pH 6.8 buffer, which yielded pure rALD/H35K.

For Western blot analysis, proteins were transferred to a PVDF membrane and were processed using standard procedures known in the art using primary monoclonal antibody to alpha-toxin mutant. Blots confirmed the presence of rALD/H35K antigen with a band roughly at ~32 kDa. In addition, N-terminal sequencing of rALD/H35K confirmed the presence of the *hla-ALD*/*H35K* gene product.

Alpha-toxin mutants rALD and rH35K were purified using the same methodology.

### Example 8. Production of Alpha-Toxin, rALD/H35K Polyclonal Antibodies

The rALD/H35K (50 µg) was injected into New Zealand White rabbits with adjuvant (CFA followed by IFA) at a 1:1 ratio 3 times, 2 weeks apart. rALD/H35K antiserum recognized rALD/H35K and native *S. aureus* alpha-toxin (List Biological Laboratories) as an identical antigen in an immunodiffusion assay against the antigen. rALD/H35K antiserum recognized both wild type and mutant alpha-toxin, as shown by Western blot and ELISA. These results indicate that the rALD/H35K vaccine generated antibodies reactive with native alpha-toxin.

Positive bleeds were combined and IgGs were purified on a protein G column. Purified anti-ALD/H35K IgG was then used in animal models.

### Example 9. Immunochemical Analysis of Alpha-Toxin Antigens

Double immunodiffusion in 1% agarose gel was carried out to determine the specificity of the rALD/H35K antisera, as well as to determine the antigenicity of alpha-toxin antigens. Briefly, 10 µl/well of 200 µg/ml each alpha-toxin antigen (outside wells) and 10 µl/well of rALD/H35K antiserum (center well) was allowed to diffuse through the gel overnight in a humid environment. The agarose gel was then washed in PBS and pressed, dried and stained with Coomassie blue. The gels were analyzed for precipitin bands, which are formed when antigen and antibody bind together to form an antibody-antigen complex. When two antigens, which have shared epitopes that react to an antiserum, are placed into adjacent wells and diffuse against the same antiserum, their precipitin lines will fuse together forming a "line of identity". A partial line of identity (a spur at the meeting point of two precipitin lines) between two antigens is formed when not all epitopes reacting with Abs from the antiserum are present in both antigens.

Each of four proteins, native alpha-toxin purified from *S. aureus* (List Biological Laboratories), and recombinant mutants rALD/H35K, rALD and rH35K, reacted with anti-rALD/H35K sera as a single precipitin band forming a line of identity indicating that antiserum raised against rALD/H35K recognizes native *S*. *aureus* alpha-toxin, and the mutants rALD and rH35K and rALD/H35K as identical or very similar antigens. Figure 1.

### Example 10. Alpha-Toxoid Hybridoma Production

BALB/c mice were immunized with rALD. Immunized splenocytes were collected from mice in this study and fused to Sp2/O myeloma cells, using 50% polyethylene glycol. The fused cells were resuspended in a selection medium, seeded into 96-well tissue culture plates and incubated under humidified conditions in a 37°C incubator with 8% CO2. Supernatants of growing cultures were screened on ELISA plates coated with purified rALD antigen, for monoclonal antibody (MAb) secretors. Several hybridomas were generated and 11 MAb secretors were established after 2 sequential cloning processes. Seed stocks were generated from mass cultures established from these clones that were also used to produce mouse ascites fluid from which purified MAbs were prepared and further characterized.

### Example 11. Characterization of Alpha-Toxin Monoclonal Antibodies

Characterization analyses revealed that 9 of 11 established alpha-toxin (Alt) MAbs prepared as described above bind specifically to native *S*. *aureus* alpha-toxin (List Biological Laboratories) in Western blot evaluation, where as 2 of 11 did not recognize native alpha-toxin. All MAbs that were Western blot positive neutralized wild type alpha-toxin in red blood cell (RBC) hemolytic experiments (See Example 15). Isotyping evaluation revealed all 11 established MAbs are of the IgG1 kappa sub-class. Seed stocks were generated from mass cultures of established clones that were also used to produce mouse ascites fluid from which purified MAbs were prepared and further characterized.

### Example 12. In Vitro Determination of Cytotoxicity Activity by Hemolytic Assay

A 1.0 µg/mL solution of purified alpha-toxin was prepared in a 10 mM Tris-HCl solution that contains 0.85% sodium chloride (NaCl), pH 7.2 (dilution/wash buffer). Serial 2-fold dilutions of alpha-toxin antigens were performed on a 96 well plate. Cell control wells that contain wash buffer only (no alpha-toxin), were included on each assay plate. Rabbit RBCs (Colorado Serum Co., cat# CS 1081) were sequentially washed 2 times at 10 volumes per wash before re-adjustment to the initial concentration with wash buffer. An equal volume of RBC suspension was added to each well that contain alpha-toxin and wash buffer. The plate was incubated at 37°C for 30 minutes to allow alpha-toxin to lyse the RBCs. The plate was then centrifuged to pellet all RBCs and cell debris before a dilution of each supernatant was performed in wash buffer in corresponding wells of another polystyrene ELISA plate. Optical densities (OD) of the supernatants were measured at 450 nm with the aid of an ELISA plate reader that subtracts the cell control (no toxin) OD as background before reporting data. The percent of RBCs that were lysed due to the alpha-toxin activity was then calculated.

Complete or nearly 100% hemolysis was observed with 0.5 µg/mL of native *S*. *aureus* alpha-toxin or 0.5 µg/mL wild type recombinant alpha-toxin (Table 5). However, no measurable hemolytic activity was detected with >185 times more rALD/H35K antigen (92.8 µg/mL) in this assay. These results demonstrate that rALD/H35K is non-hemolytic *in vitro* and thus rALD/H35K could be used as a vaccine to generate antibodies that are reactive with native alpha-toxin from *S*. *aureus.*

**Table 5**

| Hemolytic activity of rALD/H35K as compared to native and recombinant wild type Alpha-Toxin | | |
|---|---|---|
| **Sample Description** | **Concentration (µg/ml)** | **% Hemolysis** |
| Native *S*. *aureus* | 0.5 | 100 |
| Alpha-toxin | | |
| Wild Type Recombinant | 0.5 | 97.0 |
| Alpha-toxin | 0.03 | 0.4 |
| Alpha-Toxin Mutant | 92.8 | 0 |
| rALD/H35K | | |

### Example 13.

### Polyclonal Antibody Neutralization of S. aureus Alpha-toxin Hemolytic Activity

Serial two-fold dilutions of the rabbit serum antibodies (anti-rALD/H35K from 4 different rabbits) or normal rabbit serum were performed on a 96 well assay plate. Cell control wells that contain wash buffer only (no alpha-toxin and no antibodies) and alpha-toxin control wells (no antibodies), were included on each assay plate. An equal volume of 4x concentrated alpha-toxin (2 µg/mL) in wash buffer is added to all wells with antibody and those with wash buffer only for toxin positive control. Wash buffer at equal volume was added to all cell control wells that contain wash buffer only. To each well with diluted antibody, alpha-toxin and wash buffer for cell control, was added washed RBCs in a volume equal to that in each well. As a result, all antibody and toxin concentrations are diluted 4 times that of starting concentrations. The plate was incubated in a humidified 37°C incubator for 30 minutes. The plate was then centrifuged to pellet all RBCs and cell debris before a dilution of each supernatant was performed in wash buffer in corresponding wells of another polystyrene ELISA plate. Optical densities (OD) of the supernatants were measured at 450 nm with the aid of an ELISA plate reader that subtracts the cell control (no toxin) OD as background before reporting data. The neutralization capacity of each antibody was determined relative to the alpha-toxin positive control.

Results (set forth in Table 6) demonstrate that all 4 rabbits that were immunized with rALD/H35K produced neutralizing antibodies to native alpha-toxin from *S*. *aureus.* Anti-ALD/H35K hyper-immune sera were able to neutralize roughly 50% at approximately 1:2648 to 1:6125 dilution, whereas normal rabbit sera was not able to neutralize 50% of alpha-toxin hemolytic activity with 25-fold more concentrated sera at a 1:100 dilution. These data clearly demonstrate that ALD/H35K-specific antibodies are effective in neutralizing native alpha-toxin activity *in vitro.*

**Table 6**

| *In Vitro* neutralization of alpha-toxin hemolytic activity by polyclonal sera | | | | | |
|---|---|---|---|---|---|
| Dilution Factor of Sera | Percent Neutralization by | | | | |
| | Anti-rALD/H35K Rabbit # 76b | Anti-rALD/H35K Rabbit # 76a | Anti-rALD/H35K Rabbit # 77a | Anti-rALD/H35K Rabbit # 77b | Normal Rabbit Serum |
| 100 | 100.8 | 101.0 | 101.1 | 101.3 | 0.1 |
| 200 | 99.9 | 100.3 | 100.2 | 100.5 | -6.8 |
| 400 | 100.2 | 100.3 | 100.3 | 100.4 | -5.5 |
| 800 | 100.0 | 100.2 | 100.0 | 100.3 | -0.9 |
| 1600 | 99.8 | 95.7 | 85.7 | 99.9 | -1.7 |
| 3200 | 80.3 | 61.4 | 47.0 | 82.3 | 3.0 |
| 6400 | 38.3 | 29.9 | 29.4 | 47.0 | 6.0 |
| 12800 | 29.9 | 29.5 | 28.4 | 30.6 | 16.2 |
| 25600 | 13.8 | 11.7 | 9.5 | 10.6 | 11.8 |
| 51200 | 12.2 | 10.5 | 13.6 | 11.4 | 13.5 |
| 102400 | 6.8 | 5.3 | 7.5 | 4.9 | 10.7 |
| 204800 | 14.5 | 8.8 | 13.3 | -3.6 | 11.2 |
| Dilution Factor at 50% Neutralization | 5506 | 4355 | 2648 | 6125 | Not measurable <<100 |

### Example 14.

### Immunogenicity of Alpha-Toxin Antigens and Reactivity of Anti-rALD/H35K and Neutralization Activity with Native S. aureus Alpha-Toxin

Polyclonal hyper-immune mouse sera were was prepared by immunizing 10 mice per group. Mice were given 3 injections, 2 weeks apart, with 2.5 µg of antigen (rALD/H35K, rH35K, rH35R, or rALD), with or without alum as an adjuvant. Mice were exsanguinated 1 week after the last injection, mouse sera for the respective antigens were pooled, and standard quantitative ELISA was carried out to determine the IgG titers to wild type alpha-toxin and to the homologous antigen. All mouse sera pools recognized the homologous antigen and native alpha-toxin from *S*. *aureus.* These results demonstrate that the double mutant rALD/H35K was able to produce higher levels of alpha-toxin IgGs as compared to other single point mutation antigens, and greater than or similar titers to the rALD antigen.

Mouse sera pools were also tested for neutralization of hemolytic activity caused by *S*. *aureus* alpha-toxin in vitro as described in Example 13. The results (set forth in Table 7) show that anti-rALD/H35K sera were effective in neutralizing hemolytic activity.

**Table 7**

| Immunogenicity, reactivity with native alpha-toxin, and neutralization of hemolytic activity by polyclonal mouse sera | | |
|---|---|---|
| **Mouse Serum** | **Anti-Native Alpha-Toxin IgG** | **EU at 50% Neutralization** |
| | **(EU/ml)** | |
| Anti-rALD/H35K | 100 | 0.307 |
| Anti-rALD/H35K with Alum | 189 | 0.459 |
| Anti-rH35K with Alum | 69 | 0.194 |
| Anti-rALD with Alum* | 207 | 0.396 |
| Anti-rH35R with Alum | 17 | NA |
| Anti-rH35R | 6 | NA |
| Anti-rH35K with Alum | 3 | NA |
| Anti-rH35K | 6 | NA |
| Anti-rALD with Alum* | 60 | NA |
| Anti-rALD | 45 | NA |
| *Results are report from two different mouse serum pools. | | |

### Example 15. Neutralization of Alpha-Toxin Hemolytic Activity by Monoclonal Antibodies

Tissue culture supernatants containing anti-alpha-toxin MAbs (obtained as described in Example 10) were characterized for their ability to neutralize alpha-toxin *in vitro.* As negative controls, a MAb specific to nicotine and normal rabbit serum, respectively, were evaluated for neutralizing activity.

Serial two-fold dilutions of the antibodies were performed on a 96 well assay plate. Cell control wells that contain wash buffer only (no alpha-toxin and no antibodies) and alpha-toxin control wells (no antibodies), were included on each assay plate. An equal volume of alpha-toxin (2 µg/ml) in wash buffer was added to all wells with antibody and those with wash buffer only for toxin positive control. Wash buffer at equal volume was added to all cell control wells that contain wash buffer only. To each well with diluted antibody, alpha-toxin and wash buffer for cell control, was added washed RBCs in a volume equal to that in each well. As a result, all antibody and toxin concentrations are diluted 4 times that of starting concentrations. The plate was incubated in a humidified 37°C incubator for 30 minutes. The plate was then centrifuged to pellet all RBCs and cell debris before a dilution of each supernatant was performed in wash buffer in corresponding wells of another polystyrene ELISA plate. Optical densities (OD) of the supernatant were measured at 450 nm with the aid of an ELISA plate reader that subtracts the cell control (no toxin) OD as background before reporting data. The neutralization capacity of each antibody was determined relative to the alpha-toxin positive control.

The 9 MAbs that bind to native alpha-toxin as demonstrated by Western blot analysis were shown to neutralize *in vitro* hemolytic activity by native alpha-toxin (data set forth in Table 8). The two MAbs that were negative for binding native alpha-toxin by Western blot and a non-specific monoclonal antibody (2Nic311) were negative for alpha-toxin neutralizing activity.

**Table 8**

| *In vitro* neutralization of alpha-toxin hemolytic activity by monoclonal and polyclonal antibodies | | | |
|---|---|---|---|
| **Antibody** | **MAb or Pab** | **Antibody Specificity** | **Dilution Factor at 50% Neutralization** |
| 2Nic311 | MAb | Nicotine | NA |
| 1Alt009 | MAb | Native alpha-toxin | 262 |
| 1Alt026 | MAb | Native alpha-toxin | 19 |
| 1Alt056 | MAb | Native alpha-toxin | 74 |
| 1Alt146 | MAb | Native alpha-toxin | 44 |
| 1Alt415 | MAb | rALD | NA |
| 1Alt562 | MAb | rALD | NA |
| 1Alt633 | MAb | Native alpha-toxin | 27 |
| 1Alt660 | MAb | Native alpha-toxin | 504 |
| 1Alt722 | MAb | Native alpha-toxin | 96 |
| 1Alt810 | MAb | Native alpha-toxin | 150 |
| 1Alt824 | MAb | Native alpha-toxin | 83 |

NA = no measurable neutralizing activity was detected or 50% neutralization was not achieved.

### Example 16.

### Neutralization of Alpha-Toxin Hemolytic Activity from S. aureus Cell Culture Supernatants by Polyclonal Rabbit Serum

The ability of anti-rALD/H35K antibodies to neutralize *S*. *aureus* secreted alpha-toxin was demonstrated in an *in vitro* hemolytic assay. Overnight *S*. *aureus* cultures from isolates Wood (ATCC # 10832, alpha-toxin prototype isolate) and Nabi clinical isolate MRSA 328 were adjusted to 2.0 OD₅₄₀ₙₘ, centrifuged, and the resulting supernatants were filtered. The four-fold diluted supernatants were then than added to serially diluted rabbit anti-rALD/H35K rabbit serum and incubated for 10 minutes. After the incubation, freshly obtained rabbit erythrocytes were added and plates were incubated for 30 minutes at 37°C. After the incubation, micro-titer plates were centrifuged at 2000 rpm for 10 minutes and degree of lysis was quantitated by measuring levels of released heme using an ELISA reader at wavelength of 410 nm.

The results (set forth in Table 9) demonstrate the neutralizing activity of anti-rALD/H35K serum. At a dilution of 1:1000, anti-ALD/H35K was able to completely neutralize 100 ng/mL purified native alpha-toxin and bacterial secreted alpha-toxin from two *S*. *aureus* clinical isolates *in vitro.*

**Table 9**

| Neutralization of Alpha-Toxin Hemolytic Activity from *S*. *aureus* Cell Culture Supernatants by Anti-rALD/H35K Rabbit Serum | | | |
|---|---|---|---|
| **Dilution of Anti-rALD/H35K Rabbit Serum** | **Percent Inhibition of Hemolytic Activity** | | |
| | **Native Alpha-Toxin (100 ng/mL)** | **Bacterial Cultured Supernatants** | |
| | | **Wood** | **MRSA 328** |
| 1:10 | 100 | 99 | 100 |
| 1:10² | 100 | 100 | 99 |
| 1:10³ | 100 | 99 | 98 |
| 1:10⁴ | 71 | 99 | 73 |
| 1:10⁵ | 40 | 47 | 33 |
| 1:10⁶ | 24 | 8 | 10 |
| 1:10⁷ | 16 | 15 | 26 |
| 1:10⁸ | 16 | 0 | 22 |

### Example 17.

### In vivo Neutralization of S, aureus Native Alpha-Toxin Challenge by Monoclonal and Polyclonal Antibodies

The *in vivo* neutralization efficacy of one of the alpha-toxin MAbs obtained as described in Example 10 (MAb 1Alt660) and anti-rALD/H35K polyclonal antibodies were assessed as follows. BALB/c mice were intra-peritoneally (IP) administered 100 µg of MAb 1 Alt660. As a control, another group of mice were given MAb generated against *E*. *coli* cell wall component (MAb 158). Similarly, mice were administered 500 µg total anti-ALD/H35K IgG obtained from rALD/H35K vaccinated rabbits as described in Example 8, with another group of mice administered an equivalent of normal rabbit IgG as a control. Twenty four hours later, the mice were challenged intra-dermally (ID) by 10 µg of native alpha-toxin (List Biological Laboratories), and were observed for skin lesions and lethality for seven days.

Passive immunization data showed that both monoclonal antibody 1 Alt660 and anti-ALD/H35K IgG protected against wound formation and alpha-toxin induced mortality. Results are summarized in Table 10.

**Table 10**

| *In vivo* Neutralization of Native *S*. *aureus* Alpha-Toxin Challenge in BALB/c Mice by Monoclonal and Polyclonal Antibodies | | | | |
|---|---|---|---|---|
| **Immunization (IP)** | **Toxin Challenge (ID)** | **Post-Challenge Survival (Percent Survival)** | | |
| **(Day -1)** | **(Day 0)** | **24 hrs** | **40 hrs** | **7 days** |
| MAb 1Alt660 | 10 µg Native Alpha-Toxin | 10/10 | 10/10 | 10/10 (100 %) |
| MAb 158 *(E. coli* -specific) | | 0/10 | 0/10 | 0/10 (0 %) |
| Anti-rALD/H35K IgG (500µg Total IgG) | | 10/10 | 10/10 | 10/10 (100 %) |
| Normal Rabbit IgG (500 µg Total IgG) | | 6/10 | 4/10 | 4/10 (40 %) |
| PBS | | 0/10 | 0/10 | 0/10 (0 %) |

### Example 18.

### Use of Anti-rALD/H35K IgG and StaphVAX IgG (Type 5 and Type 8 IgG) against Lethal challenge of S. aureus

To demonstrate the advantages of combining neutralizing and opsonic antibodies in a therapy against highly virulent *S*. *aureus* isolates, BALB/c mice were administered via the intraperitoneal route anti-ALD/H35K rabbit IgG (neutralizing antibodies) in combination with 200 µg of opsonic antibodies, *S*. *aureus* Type 5 and Type 8 capsular polysaccharide human antibodies (AltaStaph, Nabi Biopharmaceuticals). As controls, mice were administered an equivalent dose of antibodies comprising anti-rALD/H35K IgG or AltaStaph alone or non-immune IgG (standard human IGIV). Twenty four hours later, mice were challenged IP by 5x10⁴ CFU of *S*. *aureus* Nabi MRSA 328, which secretes high levels of alpha-toxin, in 5% hog mucin and monitored for morbidity and mortality at 24 hours, 40 hours and 5-7 days after bacterial challenge.

The results (set forth in Table 11) demonstrate the protective efficacy of the combination of *S*. *aureus* neutralizing and opsonic antibodies. Thus, mice immunized with rabbit anti-rALD/H35K IgG (neutralizing) in combination with AltaStaph (opsonizing anti-Type 5 and Type 8 capsular polysaccharide IgG) were protected from the highly virulent S. *aureus* challenge, whereas mice that received either anti-rALD/H35K IgG or AltaStaph alone did not survive the challenge.

**Table 11**

| Efficacy of Anti-rALD/H35K IgG and StaphVAX IgG against a lethal challenge of *S*. *aureus* | | | |
|---|---|---|---|
| **Immunizing Agent(s)** | **Post-Challenge Survival** | | |
| | **24** | **40** | **7 Days** |
| Anti-ALD/H35K rabbit IgG (4 mg total IgG) AltaStaph (200 µg specific T5CP IgG, ~ 7 mg total IgG) | 10/10 | 10/10 | 10/10 |
| Anti-rALD/H35K rabbit IgG (2 mg total IgG) AltaStaph (200 µg specific T5CP IgG, ~ 7 mg total IgG) | 10/10 | 9/10 | 9/10 |
| Anti-rALD/H35K rabbit IgG (1 mg total IgG) AltaStaph (200 µg specific T5CP IgG, ~ 7 mg total IgG) | 8/10 | 6/10 | 6/10 |
| Anti-rALD/H35K rabbit IgG (4 mg total IgG) | 0/10 | 0/10 | 0/10 |
| AltaStaph (200 µg specific T5CP IgG, ~ 7 mg total IgG) | 0/10 | 0/10 | 0/10 |
| Normal rabbit IgG (4 mg total IgG) | 3/10 | 3/10 | 3/10 |
| Standard human IGIV (6 µg specific T5CP IgG, ~ 7 mg total IgG) | 0/10 | 0/10 | 0/10 |
| PBS | 0/10 | 0/10 | 0/10 |

### Example 19.

### Method for preparation of conjugate vaccine using rALD/H35K as a carrier protein

A non-toxic alpha-toxin mutant, rALD/H35K, was used as a protein carrier in polysaccharide-protein conjugate vaccines. A method for conjugating *S. epidermidis* polysaccharide antigen PS1 to rALD/H35K is described.

A PS1 solution (10 mg/mL) was prepared in 0.1 M MES buffer. Adipic acid dihydrazide (ADH) was added as a dry powder to yield a final concentration of 0.2 M. To initiate the reaction, ethyl-3-(3-dimethyl aminopropyl) carbodiimide (EDC) was added to a final concentration of 0.05 M and was allowed to stir for an additional 30 min. The reaction mixture containing derivatized PS1 (PS1_{-AH}) was then dialyzed against 1 M NaCl, followed by distilled water, and then was chromatographed through a Sephadex G25 column to remove the residual salt. The amount of ADH incorporated on antigen PS1_{-AH} was determined colorimetrically by trinitrobenzene sulfonic acid (TNBS) assay.

A solution of containing rALD/H35K (2 mg/mL) was prepared in 0.05 M sodium phosphate / 0.2M imidazole buffer containing 0.3 M NaCl. Subsequently, succinic anhydride was added to the protein at w/w ratio of 2:1, and the pH was maintained at 8 using 1M NaOH for 2 hours while stirring. The derivatized carrier protein, rALD/H35K-_{suc}, was then dialyzed against 0.2M NaCl and was further purified on a Sephadex-G25 column, pooled and concentrated. Protein content was measured by BCA (Pierce) and efficiency of succinylation of protein was estimated by measuring amino groups before and after reaction by TNBS assay.

A solution containing 10 mg PS1-_{AH} and 10 mg rALD/H35K-_{suc} in 1mL of 0.1M MES / 0.2 M NaCl buffer, pH 5.7-5.8, was prepared. To the reaction mixture, EDC was added to yield a final concentration of 50 mM and the reaction was maintained for 30 min while stirring, and then was subsequently dialyzed against 0.2 M NaCl. Pure conjugate was obtained by size exclusion chromatography on Sephacryl S-300 column eluted with 0.2 M NaCl. The amount of PS1 and carrier protein (rALD/H35K) in the conjugate was determined by phosphorous assay and BCA assay (Pierce), respectively. As a control, a conjugate of PS1 and a nontoxic mutant of *Pseudomonas aeruginosa* exotoxin A (rEPA) was prepared (PS 1-rEPA) using the same methodology.

Table 12 compares the characteristics of *S*. *epidermidis* PS1-rALD/H35K to the PS1-rEPA conjugate. Succinic derivatives of rALD/H35K and rEPA had very similar characteristics in terms of efficiency of succinylation as monitored via reduction in number of amino groups, which was 80% and 75%, respectively. The resultant conjugates PS1-rALD/H35K and PS 1-rEPA had similar w/w PS/PR ratios (0.71 and 0.61).

**Table 12**

| Characterization of *S*. *epidermidis* PS1-conjugates prepared with rALD/H35K or rEPA as a carrier protein | | | | | |
|---|---|---|---|---|---|
| **Type of conjugate** | **Derivatives of PS or PR** | | **PS in the conjugate µg/mL** | **PR in the conjugate µg/mL** | **PS/PR ww** |
| | Amount of Hydrazide in PS1 (w/w) | Reduction in number of NH₂ groups in Protein | | | |
| PS1-rALD/H35K | 0.017 | 80% | 363 | 511 | 0.71 |
| PS1-rEPA | 0.017 | 75% | 279 | 454 | 0.61 |

### Example 20.

### Immunogenicity of S. epidermidis PS1-rALD/H35K Conjugate Vaccine

To evaluate the immunogenicity of PS1-rALD/H35K, 10 BALB/c mice per group were immunized 3 times, two weeks apart, with 2.5 and 10 µg PS1-rALD/H35K conjugate, with and without adjuvant (QS-21). Seven days following the third injection, mice were exsanguinated and sera collected.

The anti-PS 1 IgG and anti-alpha-toxin IgG response was measured in sera samples via ELISA using PS1 or native alpha-toxin (List Biological Laboratories) as a coating antigen, respectively. Immunogenicity results are presented as the group geometric mean (GM) values of serum IgG expressed in ELISA units/mL (EU/mL). Anti-PS 1 IgG titers were compared to the reference serum arbitrarily assigned 100 EU/mL. The titer of 100 EU/mL represents the concentration of specific IgG that gives OD₄₅₀ of 2.0 at the dilution of 1:2000 in ELISA. Anti-alpha-toxin IgG titers were calculated by interpolation to the reference serum arbitrarily assigned 5,000 EU/mL. The titer of 5,000 EU/mL represents the concentration of specific IgG that gives OD₄₅₀ of 2.0 at the dilution of 1: 5,000 in ELISA.

All serum samples were evaluated for neutralization of native alpha-toxin hemolytic activity *in vitro* as described in Example 13.

Following immunization, both anti-PS 1 IgG as well as anti-alpha-toxin IgG responses increased in a dose-dependent fashion and increased titers were demonstrated when an adjuvant was used during immunization. Although significant anti-alpha-toxin titers were induced by the PS1-rALD/H35K conjugate, only very high titered sera (>300 EU/mL) were able to neutralize the hemolytic activity of native alpha-toxin *in vitro.* Such relevant toxin-neutralization levels of anti-alpha-toxin IgG were induced in 90 to 100% animals that received 2.5 or 10 µg of PS1-rALD/H35K with adjuvant (QS-21), whereas without adjuvant, immunization with 10 µg conjugate induced 50% neutralization titers in 2/10 animals.

These data show that rALD/H35K can be used as a carrier protein for polysaccharide conjugates, e.g., PS1-rALD/H35K, which can be used to stimulate both high titers of PS antibodies and also alpha-toxin antibodies that are effective in neutralizing native alpha-toxin hemolytic activity.

**Table 13**

| Immunogenicity of *S*. *epidermidis* PS1-rALD/H35K Conjugate Vaccine | | | | | |
|---|---|---|---|---|---|
| **Vaccine** | **Dose of conjugate** | **GM Anti-PS1 IgG (EU/mL)** | **GM Anti-alpha-toxin IgG (EU/mL)** | **GM 50% neutralizing titer (range)** | **No of mice/ group with 50%-neutralization titer>4** |
| PS1-rALD/H35K | 2.5 µg | 37.60 | 68.05 | NA* | 4/10 |
| | | | | (<4-16) | |
| PS1-rALD/H35K + adjuvant | | 343.84 | 1112.64 | 25.44 | 7/10 |
| | | | | (<4-133) | |
| PS1-rALD/H35K | 10 µg | 50.32 | 127.17 | NA* | 2/10 |
| | | | | (<4-109) | |
| PS1-rALD/H35K + adjuvant | | 515.01 | 3772.52 | 50.20 | 8/10 |
| | | | | (4-252) | |

| | | | | | |
|---|---|---|---|---|---|
| GM = geometric mean *GM values were not calculated if more than 6 sera in group of 10 did not reach measurable 50% neutralization titer. The lowest 50% neutralization titer is "4" and corresponds to use of neat (undiluted serum) in the neutralization assay | | | | | |

### Example 21.

### Production of Opsonic Anti-Txpe 5, Anti-Type 8 and Anti-336) and Neutralizing (Anti-LukS-PV and Anti-ALD/H35K Polyclonal Antibodies

Antigens were injected into New Zealand White rabbits 5-6 times, 2 weeks apart. Rabbits received (1) 50 µg each Type 5-rEPA, Type 8-rEPA and 336-rEPA conjugates, (2) 50 µg each rALD/H35K and rLukS-PV with adjuvant (5% Titermax) at a 1:1 ratio, (3) 50 µg each Type 5-rEPA, Type 8-rEPA and 336-rEPA conjugates, and 50 µg each rALD/H3SK and rLukS-PV with adjuvant (5% Titermax) at a 1:1 ratio, or (4) PBS with adjuvant (5% Titermax) at a 1:1 ratio.

The antiserum generated by immunizing rabbits with Type 5-rEPA, Type 8-rEPA and 336-rEPA conjugates recognized Type 5, Type 8 and 336 polysaccharides in ELISA and immunodiffusion. Antiserum generated by immunizing rabbits with rALD/H35K and rLukS-PV recognized native *S*. *aureus* alpha-toxin (List Biological Laboratories) and rLukS-PV in ELISA and immunodiffusion. The antiserum generated by immunizing rabbits with Type 5-rEPA, Type 8-rEPA and 336-rEPA conjugates and rALD/H35K and rLukS-PV recognized Type 5, Type 8 and 336 polysaccharides, alpha-toxin and LukS-PV in ELISA and immunodiffusion.

Positive bleeds were combined and IgGs were purified on a protein G or A column. The following purified IgGs were then used in animal model experiments: (1) anti-Type 5 and anti-Type 8 capsular polysaccharide IgG and anti-336 IgG (opsonic antibodies); (2) anti-alpha-toxin ALD/H35K and anti-LukS-PV IgG (neutralizing antibodies); (3) anti-Type 5 and anti-Type 8 capsular polysaccharide IgG, anti-336 IgG, anti-alpha-toxin ALD/H35K IgG and anti-LukS-PV IgG (opsonic and neutralizing antibodies).

### Example 22.

### Protection Against S. Aureus Challenge by Active Immunization with Type 5-Conjugate. Type 8-Conjugate, 336-Conjusate, rALD/H35K and rLukS-PV

The ability of vaccines comprising Type 5-conjugate, Type 8-conjugate, 336-conjugate, rALD/H35K and rLukS-PV to protect against S. aureus-induced skin infections was assessed. New Zealand female rabbits, 5-6 month old, were immunized as described in Example 21 to generate high levels of antibodies. Rabbits were bled seven days after the 5th or 6th injection and were evaluated for Type 5, Type 8, and 336 polysaccharide, and alpha-toxin and LukS-PV IgG antibody titers by ELISA. In all relevant sera, titers for these antigens were 1:10⁵ to 10⁶ dilution for an OD₄₅₀ₙₘ = 2.0.

The rabbits' backs were shaved and intradermally injected with 10⁸ CFU/100 □L of *S*. *aureus* strains, USA300-01114 (PVL producing CA-MRSA). Animals were observed for formation of dermonecrotic lesions.

Vaccination with Type 5-rEPA, Type 8-rEPA and 336-rEPA conjugates, rALD/H35K and rLukS-PV induced high antibody titers for each subunit, respectively (dilution 1:10⁵ to 10⁶ for an OD₄₅₀ₙₘ = 2). These antibodies showed protection against abscess formation resulting from a PVL producing *S*. *aureus* isolate (or CA-MRSA USA300). That is, at the injection site, only a slight redness was observed on rabbits immunized with the pentavalent combination (Type 5-rEPA, Type 8-rEPA and 336-rEPA conjugates, rALD/H35K and rLukS-PV). In contrast, abscess formation was observed on a control rabbit, which received placebo (PBS plus Titermax). The rabbit immunized with all five antigens (Type 5-rEPA, Type 8-rEPA and 336-rEPA conjugates, rALD/H35K and rLukS-PV) was healthy on day 8. However, the rabbit immunized with placebo was observed to have clinical signs of morbidity (weight loss, lethargy). Thus, immunization with a pentavalent *S*. *aureus* vaccine containing Type 5-conjugate, Type 8-conjugate, 336-conjugate, rALD/H35K and rLukS-PV prevents *S*. *aureus* infections, including infections induced by highly invasive PVL producing strains.

### Example 23.

### Synergy of Type 5/Type 8/336 IgG (Opsonic IgG) and Alpha-Toxin/PVL IgG (Neutralizing IgG) Against S. aureus Challenge

To demonstrate the advantages of a combination of opsonic and neutralizing polyclonal antibodies (pAbs) against highly virulent *S*. *aureus* isolates, BALB/c mice were passively immunized intraperitonealy with (1) anti-Type 5 and anti-Type 8 capsular polysaccharide IgG and anti-336 IgG opsonic pAbs; (2) anti-alpha-toxin ALD/H35K and anti-LukS-PV rabbit IgG neutralizing pAbs; or (3) anti-Type 5 and anti-Type 8 capsular polysaccharide IgG, anti-336 IgG, anti-ALD/H35K IgG and anti-LukS-PV IgG (e.g., a combination of opsonic and neutralizing pAbs). As controls, mice were administered an equivalent dose, 2 mg total IgG of normal Rabbit IgG. Twenty four hours later, mice were shaved to remove the fur on their backs and were challenged via intradermal (ID) route by 1x10⁸ CFU of *S*. *aureus* USA300-01114, a CA-MRSA strain which secretes PVL and alpha-toxin. Mice were observed for dermonecrotic lesions at 16 and 72 hours.

The results (set forth in Table 14) demonstrate the protective efficacy of the combination of *S*. *aureus* neutralizing and opsonic pAbs. Thus, mice immunized with the opsonizing and neutralizing pAbs (anti-Type 5, anti-Type 8, anti-336, anti-alpha-toxin rALD/H35K and anti-LukS-PV) were protected from the highly virulent *S*. *aureus* challenge, whereas mice that received either the opsonic pAbs alone (anti-Type 5, anti-Type 8 and anti-336) or neutralizing pAbs alone (anti-alpha-toxin ALD/H35K and anti-LukS-PV) were not protected from this challenge.

**Table 14**

| Synergy of Opsonic pAbs and Neutralizing pAbs in Protection Against *S*. *Aurues* Infection | | |
|---|---|---|
| **Immunizing Agent(s)** | **Post-Challenge Number of Mice with Dermonecrotic Lesions** | |
| | **16 Hours** | **72 Hours** |
| Anti-Type 5, Anti-Type 8, Anti-336 rabbit IgG (opsonic pAbs, 1 mg total IgG) and | 0/10 | 1/10 |
| Anti-alpha-toxin rALD/H35K and Anti-LukS-PV rabbit IgG (neutralizing pAbs, 1 mg total IgG) | | |
| Anti-Type 5, Anti-Type 8, Anti-336 rabbit IgG (Opsonic pAbs, 1 mg total IgG) and | 0/10 | 5/10 |
| Normal Rabbit IgG (1 mg total IgG) | | |
| Anti- alpha-toxin rALD/H35K and Anti-LukS-PV rabbit IgG (neutralizing pAbs, 1 mg total IgG) and | 0/10 | 4/10 |
| Normal Rabbit IgG (1 mg total IgG) | | |
| Normal rabbit IgG (2 mg total IgG) | 0/10 | 9/10 |
| PBS - Placebo | 0/10 | 10/10 |

### Example 24.

### Cloning, Expression and Purification of S. aureus Gamma-Hemolysin Subunits

Genomic DNA was extracted from *S*. *aureus* strain ATCC 49775, and primers were designed to clone the gamma-hemolysin genes, *hlgA, hlgB, and hlgC,* in pTrcHisA plasmid vector which confers ampicillin resistance. Using polymerase chain reaction (PCR), the signal peptide was removed from all three genes, and the BamHI and NcoI site were engineered for cloning. The PCR product was digested with BamHI and NcoI, and the three genes were ligated each separately with the similarly digested vector. The ligated DNA was then transformed into GC-10 *E*. *coli* chemically competent cells which were grown on LB agar plates containing ampicillin. PCR was used to screen colonies for the right gene insert, and the positive colonies were grown in LB broth, after which the plasmid DNA was extracted and digested with BamHI and NcoI for confirmation. Samples with the right gene insert were then sequenced, and plasmids with the correct inserts were then transformed into *E*. *coli* BL21 (DE3) pLysS chemically competent cells for protein expression. The same approach was used to purify HlgA, HlgB, and HlgC by growing *E*. *coli* expressing each of the subunits separately in 2 L of Circlegrow media at 37° C, followed by induction with IPTG at 30° C. The bacterial cells were then harvested by centrifugation and the cell paste was exposed to an osmotic shock using a 20% sucrose solution followed by resuspension in a hypo-osmotic buffer. The cell debris was removed by centrifugation and the supernatant was filtered and then loaded on an SP Sepharose cation exchange column. A linear gradient to of sodium chloride solution was used for elution, and the eluted samples were analyzed on an SDS-PAGE. The samples containing the right size protein were pooled and loaded on a ceramic hydroxyapatite (CHT) column, and a linear gradient of sodium chloride was again used for elution.

The samples were analyzed by SDS-PAGE and western blot. For Western blot analysis, proteins were transferred to a PVDF membrane and were processed using standard procedures known in the art using anti-LukS-PV or anti-LukF-PV antibodies. Blots confirmed the presence of rHlgA, rHIgB, and rHlgC antigens with a band roughly at ~32-34 kDa.

### Example 25.

### Production and characterization of Leukocidin Polyclonal Antibodies

rLukS-PV, rLukF-PV, rHlgA, rHlgB, or rHlgC (50 µg each) were injected into New Zealand White rabbits with adjuvant (Sigma Titermax or CFA followed by IFA) at a 1:1 ratio, 3 to 6 times, 2 weeks apart. LukS-PV antiserum recognized rLukS-PV as an identical antigen in an immunodiffusion assay against the antigen, while rLukF-PV antiserum recognized LukF-PV. rLukS-PV or rLukF-PV did not react with the heterologous antisera.

### Example 26

### Cross-reactivity of S. aureus Leukocidin Subunits. HlgA, HlgB. HlgC with PVL Antibodies

Double immunodiffusion in 1% agarose gel was carried out to determine the specificity of the PVL antisera, as well as to determine the antigenicity of Hlg subunit antigens. Briefly, 10 µl/well of 200 µg/ml each leukocidin antigen (outside wells) and 10 µl/well of LukS-PV antiserum or LukF-PV antiserum (center well) was allowed to diffuse through the gel overnight in a humid environment. The agarose gel was then washed in PBS and pressed, dried and stained with Coomassie blue.

The gels were analyzed for precipitin bands, which are formed when antigen and antibody bind together to form an antibody-antigen complex. When two antigens, which have shared epitopes that react to an antiserum, are placed into adjacent wells and diffuse against the same antiserum, their precipitin lines will fuse together forming a "line of identity". A partial line of identity (a spur at the meeting point of two precipitin lines) between two antigens is formed when not all epitopes reacting with Abs from the antiserum are present in both antigens.

Three *S. aureus* leukocidin S subunits, rHlgA (A), rHlgC and LukS-PV (S) reacted with anti-LukS-PV sera as a single precipitin band forming a line of partial identity and these S subunits did not react with anti-LukF-VP antiserum. This indicates that antiserum raised against rLukS-PV recognizes *S aureus* gamma-toxin S subunits, HlgA and HlgC, as similar antigens having not all but some shared epitopes. Similarly, two leukocidin F subunits, rHIgB (B) and rLukF-PV (F), reacted with anti-LukF-PV sera as a single precipitin band forming a line of partial identity and did not react with LukS-PV antiserum. This indicates that antiserum raised against rLukF-PV recognizes *S*. *aureus* gamma-hemolysin F subunit, HlgB, as similar antigens having not all but some shared epitopes. Thus, PVL antibodies are cross-reactive to *S*. *aureus* leukocidin, gamma-hemolysin.

Quantitative ELISA was performed with both anti-LukS-PV and anti-LukF-PV antibodies, confirming that there is cross-reactivity among the leukocidin S subunits (rHlgA, rHlgC and rLukS-PV), as well as cross-reactivity among the leukocidin F subunits (rHlgB, and rLukF-PV). No cross-reactivity between leukocidin subclasses S and F was demonstrated.

### Example 27.

### Reactivity of Leukocidin Antibodies

Rabbit polyclonal antibodies (anti-LukS-PV, anti-LukF-PV, anti-HlgA, anti-HlgB and anti-HlgC), were evaluated for reactivity with various leukocidin antigens, including rLukS-PV, rLukF-PV, rHlgA, rHlgB and rHlgC, using standard ELISA techniques. Briefly, 96-well plates were coated with 1 µg/mL of the specific leukocidin antigen, and then plates washed and then blocked with BSA. Plates were washed again, and then anti-leukocidin rabbit sera or reference control rabbit sera were serially diluted down the plates and incubated. Plates were washed again and a secondary antibody conjugated to horse radish peroxidase (HRP) was added. Plates were washed and then developed using a peroxidase substrate system with reactivity determined by reading at OD 450 nm. Cross-reactivity was considered positive when OD at 450 nm was greater than 0.2.

ELISA data demonstrate that the leukocidin S subunits (rLukS-PV, rHlgA and rHlgC) are reactive with anti-LukS-PV, anti-HlgA and anti-HlgC antibodies, while the leukocidin F subunits (rLukF-PV and rHlgB) are reactive with anti-LukF-PV and anti-HlgB antibodies (Table 15). Leukocidin S subunits (rLukS-PV, rHlgA and rHlgC) were not reactive with anti-HlgB and anti LukF-PV antibodies, while leukocidin F subunits (rLukF-PV and rHlgB) were not reactive with anti-LukS-PV, anti-HlgA, or anti-HlgC antibodies. Thus, the leukocidin S antibodies are cross-reactive with heterologous leukocidin S subunits and leukocidin F antibodies are cross-reactive with heterologous leukocidin F subunits.

**Table 15**

| Cross-reactivity of leukocidin antibodies with leukocidin subunits | | | | | |
|---|---|---|---|---|---|
| | OD at 450 nm for Anti-Serum Dilution of 1:1000 | | | | |
| Coating Antigen | Anti-HlgA | Anti-HlgB | Anti-HlgC | Anti-LukS-PV | Anti-LukF-PV |
| rHlgA | 3.87 | 0.08 | 1.47 | 3.5 | 0.06 |
| rHlgB | 0.06 | 2.52 | 0.14 | 0.06 | 3.52 |
| rHlgC | 0.35 | 0.18 | 1.95 | 3.43 | 0.07 |
| rLukS-PV | 1.64 | 0.1 | 1.96 | 3.7 | 0.1 |
| rLukF-PV | 0.12 | 0.38 | 0.08 | 0.1 | 3.4 |

### Example 28.

### Neutralization of S. aureus Gamma-Hemolysin by Leukocidin Polyclonal Antibodies

HL-60 cells were grown in DMEM media supplemented with 10% fetal bovine serum (FBS) in the presence of DMSO for 7 days to induce differentiation. The cells were then harvested by slow speed centrifugation, and were then seeded in a 96-well plate at 5x10⁵ cells/well using FBS free media. Different concentrations of gamma-hemolysin (HlgA/HlgB or HlgC/HlgB were incubated with different dilutions of rabbit polyclonal anti-LukS-PV antibodies, anti-LukF-PV antibodies or normal rabbit serum for 30 min at 37° C. The mixtures of the antibodies and the toxins were then added to the cells, and allowed to incubate for 24 h. XTT (2,3-Bis(2-methoxy-4-nitro-5-sulfophenyl)-2H-tetrazolium-5-carboxanilide inner salt) solution was then added to the cells and the absorbance at 450 nm was measured to determine cell viability. As a control, the reaction was carried out without the addition of any rabbit serum.

HlgA/HlgB and HlgC/B were cytotoxic to HL-60 cells at a concentration of 250 ng/mL and 32 µg/mL, respectively. Both anti-LukS-PV and anti-LukF-PV antisera were able to neutralize cytotoxicity of HglA/HlgB and HlgC/HlgB. The neutralization effect of the anti-sera was dilution dependent, in contrast to the effect of the normal rabbit sera which showed a low level of non-specific neutralization. HlgA/HlgB cytotoxic activity was neutralized by 84% and 74%, respectively by anti-LukF-PV and anti-LukS-PV antiserum at a dilution of 1:20. HlgC/HlgB cytotoxicity was neutralized by 91 % and 72%, respectively by anti-LukF-PV and anti-LukS-PV at a dilution of 1:5. This clearly demonstrates that anti-leukocidin antibodies are cross-neutralizing to the heterologous leukocidins.

**Table 16**

| Cross-neutralization of gamma-hemolysin HlgA/HlgB and HlgC/HlgB with anti-LukS-PV and anti-LukF-PV antibodies | | | |
|---|---|---|---|
| Anti-Serum Dilution | % Neutralization of HlgA/HlgB Cytotoxicity | | |
| | Normal Rabbit Serum | Anti-LukS-PV | Anti-LukF-PV |
| 1:20 | 22 | 74 | 84 |
| 1:40 | 21 | 63 | 65 |
| 1:80 | 17 | 48 | 66 |
| 1:160 | 23 | 26 | 38 |
| 1:320 | 23 | 24 | 17 |
| 1:1000 | 23 | 12 | 13 |
| 1:5 | 13 | 72 | 91 |
| 1:10 | 16 | 50 | 53 |
| 1:20 | 13 | 28 | 28 |
| 1:40 | 16 | 9 | 19 |
| 1:80 | 6 | 13 | 13 |
| 1:160 | 9 | 9 | 13 |

### Example 29.

### Neutralization of S. aureus PVL Cytotoxicity by Leukocidin Polyclonal Antibodies

HL-60 cells were grown in DMEM media supplemented with 10% fetal bovine serum (FBS) in the presence of DMSO for 7 days to induce differentiation. The cells were then harvested by slow speed centrifugation, and were then seeded in a 96-well plate at 5x10⁵ cells/well using FBS free media. PVL (32 µg/mL of rLukS-PV and rLukF-PV) were incubated with different dilutions of rabbit polyclonal anti-HlgA, anti-HlgB, and anti-HIgC antibodies or normal rabbit serum for 30 min at 37° C. The mixtures of the antibodies and the toxins were then added to the cells, and allowed to incubate for 24 h. XTT (2,3-Bis(2-methoxy-4-nitro-5-sulfophenyl)-2H-tetrazolium-5-carboxanilide inner salt) solution was then added to the cells and the absorbance at 450 nm was measured to determine cell viability. As a control, the reaction was carried out without the addition of any rabbit serum.

PVL was cytotoxic to HL-60 cells at a concentration of 32 µg/mL. Rabbit anti-HlgA, anti-HlgB and anti-HlgC antisera were able to neutralize cytotoxicity of PVL. The neutralization effect of the anti-sera was dilution dependent, in contrast to the effect of the normal rabbit sera, which showed a low level of non-specific neutralization. When the sera were diluted 1:5, PVL cytotoxic activity was neutralized 32%, 26 and 26% by anti-HlgA, anti-HlgB and anti-HlgC antiserum, respectively. Normal rabbit serum showed very little or no neutralization activity (1%) at 1:5 dilution. Thus, leukocidin-specific antibodies were demonstrated to neutralize the heterologous leukocidins.

**Table 17**

| Cross-neutralization of PVL toxin with anti-HlgA, anti-HlgB and anti-HlgC antibodies | | | | |
|---|---|---|---|---|
| Anti-Serum Dilution | % Neutralization | | | |
| | Normal Rabbit Serum | Anti-HlgA | Anti-HlgB | Anti-HlgC |
| None | 0 | 0 | 0 | 0 |
| 1:5 | 1 | 32 | 26 | 26 |

### Example 30.

### Neutralization of S. aureus Leukocidin Cytotoxicity by Leukocidin Polyclonal Antibodies

Leukocidin cytotoxicity and neutralization of leukocidin cytotoxicity by leukocidin antibodies was demonstrated. Leukocidins (either PVL or gamma-hemolysin HlgC/HlgB, each at 400 ng/ml), were incubated with different dilutions of rabbit polyclonal anti-HlgA, anti-HlgB, or anti-HlgC antibodies or normal rabbit serum for 30 minutes at 37° C. The antibody/toxin mixtures were then added to 5 X 10⁵ cells/well human polymorphonuclear leukocytes (PMNs) using FBS free media and allowed to incubate for 2 hours. XTT (2,3-Bis(2-methoxy-4-nitro-5-sulfophenyl)-2H-tetrazolium-5-carboxanilide inner salt) solution was added to the cells and the absorbance at 450 nm was measured to determine cell viability. As a control, the reaction was carried out without the addition of any rabbit serum.

*S. aureus* PVL and gamma-hemolysin (HlgC/HlgB) were cytotoxic to PMNs at a concentration of 400 ng/ml. Rabbit anti-HlgA, anti-HlgB and anti-HlgC antisera were able to neutralize PVL cytotoxicity, and anti-LukS-PV and anti-LukF-PV were able to neutralize HlgC/HlgB cytotoxicity. The neutralization effect of the anti-sera was dilution dependent, in contrast to the effect of the normal rabbit sera, which showed a low level of non-specific neutralization. These data show that leukocidin-specific antibodies are able to neutralize heterologous leukocidins.

**Table 18.**

| **Cross-neutralization *of S. aureus* leukocidins, PVL and gamma-hemolysin, with leukocidin antibodies** | | | | |
|---|---|---|---|---|
| Anti-Serum Dilution | % Neutralization of PVL Cytotoxicity | | | |
| | Normal Rabbit Serum | Anti-HlgA | Anti-HlgB | Anti-HlgC |
| None | 0 | 0 | 0 | 0 |
| 1:5 | 9 | 35 | 32 | 26 |
| 1:10 | 2 | 2 | 1 | 2 |
| None | 0 | 0 | 0 | 0 |
| 1:5 | 9 | 48 | 58 | 48 |
| 1:10 | 4 | 47 | 57 | 77 |
| 1:20 | 5 | 15 | 57 | 73 |
| 1:40 | 4 | 9 | 57 | 69 |
| 1:80 | 4 | 9 | 9 | 9 |

### Example 31

### Synergy of Type 5/Type 8/336 Monoclonal Antibodies (opsonic Antibodies) and Alpha-Toxin/Leukocidin Monoclonal Antibodies (Neutralizing Antibodies) against S. aureus

To demonstrate the advantages of combining neutralizing and opsonic monoclonal antibodies (mAbs) in a therapy against highly virulent *S*. *aureus* isolates, BALB/c mice were passively immunized intraperitonealy with (1) anti-Type 5 and anti-Type 8 capsular polysaccharide mAbs and anti-336 mAbs (opsonic mAbs), (2) anti-alpha-toxin and anti LukS-PV mAbs (toxin neutralizing mAbs), or (3) anti-Type 5 and anti-Type 8 capsular polysaccharide mAbs and anti-336 mAbs, and anti-alpha-toxin and anti-LukS-PV mAbs (combination of opsonic and neutralizing mAbs). As controls, mice were administered non-specific monoclonal antibody or PBS. Twenty four hours later, mice were shaved to remove the fur on their backs and were challenged via intradermal (ID) route by 1x10⁸ CFU of *S*. *aureus* USA300-01114, a CA-MRSA strain which secretes PVL and alpha-toxin. Mice were observed for skin and soft tissue infection at 72 hours.

The results (set forth in Table 19) demonstrate the protective efficacy of the combination of *S*. *aureus* toxin-neutralizing and opsonic antibodies at 72 hours post-bacterial challenge with *S*. *aureus* isolate USA300, as compared to the protective effect of immunization with either neutralizing or opsonic antibodies alone. Control mice that received non-specific monoclonal antibody or PBS were not protected from bacterial infection in that all mice developed necrotic skin lesions and had a higher rate of organ seeding. Mice immunized with toxin-neutralizing antibodies (anti-alpha-toxin and anti-leukocidin monoclonal antibodies) showed a reduction in the number of skin lesions, while mice immunized with opsonic antibodies (anti-Type 5, anti-Type 8, and anti-336 monoclonal antibodies) showed a reduction in organ seeding. Mice immunized with both the opsonic and neutralizing antibodies (anti-Type 5, anti-Type 8, anti-336, anti-alpha-toxin and anti-LukS-PV monoclonal antibodies) were protected from the highly virulent *S*. *aureus* challenge in that they had decreased number of skin lesions and a lower rate of organ seeding. Thus, the combination of the opsonic and toxin-neutralizing antibodies demonstrated a protective effect in preventing skin and soft tissue infection and organ seeding.

**Table 19**

| Protective Effect of Opsonic and Neutralizing Antibodies in Protection against *S*. *aureus* Challenge | | | | | |
|---|---|---|---|---|---|
| **Immunizing Agent(s)** | | **72 Hours Post-Challenge Number of Mice with** | | | |
| | | **Skin Lesions** | **Organ Seeding** | | |
| | | | **Kidneys** | **Liver** | **Lungs** |
| Opsonic Antibodies: anti-Type 5, anti-Type 8 & anti-336 | All | 6/20 (30%) | 9/20 (45%) | 7/20 (35%) | 4/20 (20%) |
| and | High dose* | 1/10 | 4/10 | 3/10 | 2/10 |
| Toxin-neutralizing antibodies: anti-alpha-toxin & anti-LukS-PV | Low dose** | 5/10 | 5/10 | 4/10 | 2/10 |
| Opsonic Antibodies: anti-Type 5, anti-Type 8 & anti-336 | All | 17/19 (89%) | 5/20 (25%) | 11/20 (55%) | 7/20 (35%) |
| | High dose* | 7/9 | 3/10 | 5/10 | 3/10 |
| | Low dose** | 10/10 | 2/10 | 6/10 | 4/10 |
| Toxin-neutralizing antibodies: 50 *µ*g IgG each of anti-alpha-toxin and anti-LukS-PV | | 3/10 (30%) | 6/10 (60%) | 5/10 (50%) | 5/10 (50%) |
| Non-specific antibody: 400 *µ*g IgG anti-*E*. *coli* | | 20/20 (100%) | 15/20 (75%) | 16/20 (80%) | 6/20 (30%) |
| PBS - Placebo (no IgG) | | 20/20 (100%) | 14/20 (70%) | 17/20 (100%) | 9/20 (45%) |

| | | | | | |
|---|---|---|---|---|---|
| *High dose: 500 µg each for opsonic antibodies and 100 µg each for neutralizing antibodies **Low dose = 100 µg each for opsonic antibodies and 50 µg each for neutralizing antibodies | | | | | |

While the invention has been described and exemplified in sufficient detail for those skilled in this art to make and use it, various alternatives, modifications, and improvements should be apparent without departing from the spirit and scope of the invention. The examples provided herein are representative, are exemplary, and are not intended as limitations on the scope of the invention. Modifications therein and other uses will occur to those skilled in the art. These modifications are encompassed within the spirit of the invention and are defined by the scope of the claims.

All patents and publications mentioned in the specification are indicative of the levels of those of ordinary skill in the art to which the invention pertains. All patents and publications are herein incorporated by reference to the same extent as if each individual publication was specifically and individually indicated to be incorporated by reference.

## Claims

1. A composition comprising a *S*. *aureus* alpha-toxin antigen and a pharmaceutically acceptable carrier, wherein said alpha-toxin antigen contains at least two alterations, relative to wild-type *S*. *aureus* alpha-toxin, that reduce its toxicity.

2. The composition of claim 1, wherein at least one of said alterations is a chemical alteration.

3. The composition of claim 2, wherein the chemical alteration is conjugation to a bacterial antigen.

4. The composition of claim 3, wherein the bacterial antigen is a *S*. *aureus* antigen.

5. The composition of claim 3, wherein the bacterial antigen is derived from bacterial species other than *S*. *aureus.*

6. The composition of claim 1, wherein at least one of said alterations is a molecular alteration.

7. The composition of claims 1, wherein at least one of said alterations is a chemical alteration and at least one of said alternations is a molecular alteration.

8. The composition of claim 6, wherein said molecular alteration is a substitution, insertion or deletion in the amino acid sequence of wild-type *S*. *aureus* alpha-toxin.

9. The composition of claim 8, wherein said molecular alteration is a substitution in the amino acid sequence of wild-type *S*. *aureus* alpha-toxin.

10. The composition of claim 9, wherein said substitution occurs at a location corresponding to His-35 of wild-type *S*. *aureus* alpha-toxin.

11. The composition of claim 10, wherein said substitution is a substitution of Arg, Lys, Ala, Leu, or Glu for His.

12. The composition of claim 9, wherein said molecular alteration is a substitution, insertion or deletion in the amino latch domain of wild-type *S*. *aureus* alpha-toxin.

13. The composition of claim 12, wherein said molecular alteration is a deletion in the amino latch domain of wild-type *S*. *aureus* alpha-toxin.

14. The composition of claim 9, wherein said molecular alteration is a deletion in the stem domain of wild-type *S*. *aureus* alpha-toxin.

15. The composition of any one of claims 1-18 for use in the treatment or prevention of *S*. *aureus* infection.
